# EUROPEAN PATENT APPLICATION

(11) **EP 2 829 274 A1**
(43) Date of publication of application: **28.01.2015**
(21) Application number: 13306080.6
(22) Date of filing: 25.07.2013
(51) Int. Cl.: A61K 35/74, C07K 14/705, C12P 21/00, C12N 15/74, C12R 1/01

(54) **Vectors for producing and secreting substance of interest by bacteria and applications thereof**

(71) Applicant: INSTITUT NATIONAL DE LA RECHERCHE AGRONOMIQUE, 75007 Paris (FR); INSTITUT NATIONAL DE LA SANTE ET DE LA RECHERCHE MEDICALE (INSERM), 75013 Paris (FR); Université de Rennes 1, 35000 Rennes (FR)
(72) Inventor: Jan, Gwenaël, 35000 Rennes (FR); Falentin, Hélène, 35310 Mordelles (FR); Halouze, Charles, 29680 Roscoff (FR); Dimanche-Boitrel, Marie-Thérèse, 35520 Melesse (FR)
(74) Representative: Regimbeau

(57) **Abstract**

The present invention relates to a vector useful for the production and secretion of a substance of interest by bacteria and applications thereof.

## Description

The present invention relates to the field of genetic engineering applicable to various industries including the pharmaceuticals, chemicals, agri-foods, cosmetics and biotechnologies industries.

In particular, the invention relates to a vector useful for the production of a substance of interest by a bacterium, particularly a bacterium belonging to the order *Actinomycetales,* more particularly to the genus *Propionibacterium,* and even more particularly to the species *Propionibacterium freudenreichii.*

Said vector can be particularly useful in the pharmaceutical field, for the large-scale production of substances whose activity is of interest in industries as diverse as pharmaceuticals, chemicals, agri-foods, cosmetics but also as a biotechnology tool for the production and secretion of substance of interest in bacteria particularly belonging to the order *Actinomycetales.*

There are today a large number of methods and means for the large-scale production of substances including peptides and proteins. With traditional chemical synthesis, which is hardly suitable for large-scale production of proteins comprising several dozen amino acids, the generally preferred method is synthesis *in vivo,* that is, in living biological systems. Many commercial societies have made a principal business of said production, and thus propose technologies based on genetic engineering and biotechnology to provide industrial quantities of peptides and/or proteins, preferably active and purified. Depending on needs, it is now possible to use various living systems such as bacteria (e.g., *Escherichia coli),* yeasts (e.g., *Saccharomyces cerevisiae, Pichia pastoris),* insect cells (baculovirus, Sf9, Sf21, etc.), plant cells and mammalian cells (e.g., CHO, HEK, COS, etc.).

The inventors have previously demonstrated (WO 2011/141558) the efficiency of using propionibacteria as living systems for producing and secreting substances including recombinant peptides and proteins, particularly of eukaryotic origin.

Indeed, these bacteria have several advantages for the large-scale production of various substances. One of their major advantages is their ability to grow on low-value culture media (e.g. including by-products of dairy and sugar industries). In fact, propionibacteria are very robust and adaptable to particular media (e.g. media containing milk, milk derivatives or molasses) that are possibly hostile to the growth and development of other living systems (e.g. due to the presence in the medium of lactic acid, salt, etc.). These bacteria have also good tolerance with respect to variations, changes or disturbances of the environmental conditions likely to occur during large-scale culture operations. Moreover, propionibacteria can be described as "natural antifungals" since they naturally produce metabolites (for example, propionate) that inhibit the development of contaminating fungi. Furthermore, they are able to produce recombinant proteins of significant size (for example, proteins of more than 500 amino acids) and can even produce several different proteins simultaneously (for example, more than a dozen different proteins).

Propionibacteria are also particularly useful for applications in the pharmaceutical and dietary fields.

In fact, one of the other major advantages of propionibacteria is that they can be used as a tool for addressing or targeting substances to the intestine. Indeed, in mammals, including humans, propionibacteria are naturally able to target the intestine where their survival time can reach two weeks, compared to only two or three days for lactic bacteria, even though they are natural hosts of this environment.

Another major advantages of propionibacteria over various other bacteria proposed so far for use in anti-tumoral therapy (e.g., in WO 01/25397 in the name of Vion Pharmaceuticals, Inc. and in WO 2009/111177 in the name of Mount Sinai School of Medicine of New York University) is that propionibacteria *per se* are efficient and specific anti-tumoral agents that can safely be used in mammals, in particular in humans. This inherent property of propionibacteria can thus be further enhanced upon using propionibacteria to deliver therapeutic agents, such as drugs, to eradicate tumor cells while at the same time preventing damage to normal cells. Propionibacteria do not need to be genetically attenuated or enhanced by genomic mutations as it is the case for other bacteria such as *Clostridium, Salmonella, Listeria,* and the like. Thus, propionibacteria can be particularly useful anti-tumoral agents thanks to both their intrinsic anti-tumoral properties and their ability to efficiently, specifically and safely deliver other anti-tumoral drugs in order to kill cancer cells.

Particularly, the Inventors have previously demonstrated that *Propionibacterium freudenreichii* (Falentin *et al.,* 2010, Plos One; Genbank accession No. FN806773) has particular cytotoxic properties with respect to colon cancer cells (Jan *et al.,* 2002; Lan *et al.,* 2007). Among other notable properties, *Propionibacterium freudenreichii* adheres to colonic epithelial cells and has no toxic effect on healthy cells (Lan *et al.,* 2008).

The Inventors have recently developed new vectors allowing the production and secretion of various substances at a high level by a bacterium, particularly belonging to the order *Actinomycetales,* including the genus *Propionibacterium.*

The doses of the secreted substances are so high that in case of large-scale substances production, they do not need to be purified. Thus, these vectors provide a system, which is not expensive, quick and easy to implement and thus particularly appropriate for large-scale substances production.

In fact, unexpectedly, the Inventors have demonstrated that signal peptides of genes encoding a surface layer protein of a *Propionibacterium freudenreichii* allow the secretion into the extracellular medium of a substance of interest at a high level by a bacterium, in particular a propionibacterium. These results are really surprising since surface layer proteins are, as indicated by their name, described as proteins anchored in the surface of *Propionibacterium freudenreichii* (Lortal *et al.* 1993).

More surprisingly, the Inventors have shown that said signal peptides particularly in combination with promoters of genes encoding a surface layer protein of a *Propionibacterium freudenreichii,* allow the secretion into the extracellular medium of a substance of interest by a bacterium, in particular a propionibacterium, at a level up to 100 times greater than that obtained with other signal peptides in the same strain.

Thus, in one aspect, the invention relates to a vector comprising:
- at least one promoter operatively linked to
- at least one nucleic acid sequence encoding a signal peptide translationally fused to
- at least one nucleic acid sequence encoding a substance of interest;

■ wherein said nucleic acid sequence encoding a signal peptide is selected from the group consisting of:
   - the sequence of the signal peptide of a gene encoding a surface layer protein of a *Propionibacterium freudenreichii;* and
   - the sequences having at least 80%, more particularly at least 85%, at least 86%, at least 87%, at least 88%, at least 89%, at least 90%, at least 91%, at least 92%, at least 93%, at least 94%, at least 95%, at least 96%, at least 97%, at least 98% and even more particularly at least 99% of identity with said sequence over the entire length of said sequence;
   and/or
■ wherein the sequence of said promoter is selected from the group consisting of:
   - the sequence of the promoter of a gene encoding a surface layer protein of a *Propionibacterium freudenreichii;* and
   - the sequences having at least 80%, more particularly at least 85%, at least 86%, at least 87%, at least 88%, at least 89%, at least 90%, at least 91%, at least 92%, at least 93%, at least 94%, at least 95%, at least 96%, at least 97%, at least 98% and even more particularly at least 99% of identity with said sequence over the entire length of said sequence.

Unless defined otherwise, all technical and scientific terms used herein have the same meaning as commonly understood to one skilled in the relevant art.

For convenience, the meaning of certain terms and phrases employed in the specification, examples and claims are provided.

A vector can be a cloning or an expression vector. The vectors according to the invention can be viral vectors such as bacteriophages, or non-viral, such as plasmid vectors. A plasmid vector is a non-viral DNA molecule hosted by a cell, distinct from the natural chromosomal DNA of said host cell and capable of autonomous replication. The choice of plasmid vector and, more particularly, the origin of replication it carries thus depend on the host cell. According to the type of host cell, several copies of a plasmid vector and/or several different plasmid vectors can be hosted simultaneously. A plasmid vector according to the invention can possibly be carried by (or "integrated in" or "inserted in") the chromosome of the host cell.

A "recombinant vector" is a vector obtained by traditional molecular biology and genetic engineering techniques, in which one or more exogenous nucleotide sequences have been inserted (or cloned).

The term "nucleic acid sequence" according to the invention refers to the sequence of a nucleic acid molecule, DNA and RNA, wherein the former can be genomic DNA, plasmid DNA, recombinant DNA or complementary DNA (cDNA), for example, and the latter can be messenger RNA (mRNA), ribosomal RNA (rRNA) or transfer RNA (tRNA). Preferably, the nucleic acid sequences of the invention refer to sequences of DNA molecules.

The promoter comprised in the vector according to the invention can be a promoter suitable for the expression of said substance of interest in a bacterium, particularly a priopionibacterium.

Particularly, said promoter is a promoter for bacterial RNA polymerase, in particular for propionibacterial RNA polymerase. Said promoter can be a constitutive and/or inducible promoter well known by one skilled in the art. Preferably, said promoter is inducible. The promoter can be developmentally regulated, inducible or tissue specific, preferably inducible in the digestive tract. In particular, said promoter is a promoter of propionic origin, in particular of *P*. *freudenreichii* origin, such as the promoter of protein PF963 of *P. freudenreichii.* More particularly, said promoter is a strong propionic promoter. As noted by the Inventors, it could be advantageous to use a promoter ensuring "basic" constitutive expression which could be strengthened by induced expression under particular conditions like conditions of digestive stress (for example the digestive stress-inducible promoter of the BCCP transcarboxylase described in Herve *et al.,* 2007). In one embodiment, the sequence of said promoter is selected from the group consisting of:
- the sequence of the promoter of a gene encoding a surface layer protein of a *Propionibacterium freudenreichii;* and
- the sequences having at least 80%, more particularly at least 85%, at least 86%, at least 87%, at least 88%, at least 89%, at least 90%, at least 91%, at least 92%, at least 93%, at least 94%, at least 95%, at least 96%, at least 97%, at least 98% and even more particularly at least 99% of identity with said sequence over the entire length of said sequence.

In fact, as mentioned above, unexpectedly, the Inventors have shown that said promoters of genes encoding a surface layer protein of a *Propionibacterium freudenreichii* in combination with signal peptides of genes encoding a surface layer protein of a *Propionibacterium freudenreichii* allow the secretion into the extracellular medium of a substance of interest by a propionibacterium at a level up to 100 times greater than that obtained with other promoters and peptide signals in the same propionibacterium strain.

These surface layer proteins have notably been described in the article of Lortal *et al.* (1993).

In particular, the sequence of said promoter is selected from the group consisting of:
- the sequence of the promoter of a gene encoding a surface layer protein of a *Propionibacterium freudenreichii,* particularly of the *Propionibacterium freudenreichii* CIRM BIA 118 strain; and
- the sequences having at least 80%, more particularly at least 85%, at least 86%, at least 87%, at least 88%, at least 89%, at least 90%, at least 91%, at least 92%, at least 93%, at least 94%, at least 95%, at least 96%, at least 97%, at least 98% and even more particularly at least 99% of identity with said sequence over the entire length of said sequence;
wherein said surface layer protein is selected from the group consisting of the surface layer protein A, the surface layer protein B, the surface layer protein C, the surface layer protein D, the surface layer protein E and the surface layer protein F, in particular of the surface layer protein A.

In particular, the sequence of said promoter is selected from the group consisting of:
- the sequence of the promoter of a gene encoding the surface layer protein A of *Propionibacterium freudenreichii* CIRM BIA 118 strain; and
- the sequences having at least 80%, more particularly at least 85%, at least 86%, at least 87%, at least 88%, at least 89%, at least 90%, at least 91%, at least 92%, at least 93%, at least 94%, at least 95%, at least 96%, at least 97%, at least 98% and even more particularly at least 99% of identity with said sequence over the entire length of said sequence;

As used herein the term "CIRM BIA 118 strain" refers to the strain numbered 118 available at the International Centre for Microbial Resources dedicated to food bacteria interest ("Centre international de ressources microbiennes dédié aux bactéries d'intérêt alimentaire") located in Rennes (France).

Advantageously, the vector according to the invention can comprise at least two promoters such as defined above which, even more advantageously, will be situated preferably in a series (but not necessarily coupled to each other), in order to increase the expression level of said substance of interest ("expression booster" effect).

The recombinant vector according to the present invention comprises at least one nucleic acid sequence encoding a signal peptide translationally fused to at least one nucleic acid sequence encoding a substance of interest. In particular, said signal peptide is a secretory signal peptide. The term "secretory signal peptide" refers to any peptide allowing the secretion, in particular into the extracellular medium, of said substance of interest, by a host cell comprising the vector according to the invention, particularly by a propionibacterium. In particular, said signal peptide is a propionibacterial secretory signal peptide, allowing the secretion, in particular into the extracellular medium, of said substance of interest, by a host cell comprising the vector according to the invention, particularly by a propionibacterium. Particularly, a signal peptide of a propionibacterium selected from dairy propionibacterium and so-called "cutaneous" propionibacterium (CPB) can be used. Among other examples, the following dairy propionibacterium can be cited: *Propionibacterium freudenreichii, P. jensenii, P. thoenii P. acidipropionici* and *P. microaerophilum.* The following cutaneous propionibacterium can also be cited: *P. acnes, P. granulosum, P. avidum* and *P. propionicum.* More particularly, a signal peptide of a propionibacterium selected from the following species can be used: *Propionibacterium freudenreichii,* more particularly the subspecies *P*. *freudenreichii* subsp. *freudenreichii* and *P. freudenreichii* subsp. *shermanii,* and *Propionibacterium acnes,* wherein said propionibacterium is preferentially *P*. *freudenreichii* subsp. *shermanii.*

In one embodiment, said nucleic acid sequence encoding a signal peptide can be selected from the group consisting of:
- the sequences SEQ ID NO: 1 to 18, particularly the sequence SEQ ID NO: 18;
- the nucleic acid sequences encoding the signal peptides of sequences SEQ ID NO: 45 to 57; and
- the sequences having at least 80%, more particularly at least 85%, at least 86%, at least 87%, at least 88%, at least 89%, at least 90%, at least 91%, at least 92%, at least 93%, at least 94%, at least 95%, at least 96%, at least 97%, at least 98% and even more particularly at least 99% of identity with one of said sequences over the entire length of said sequence.

Table I below presents the nucleotide (NT) sequences, as well as the corresponding amino acid (AA) sequences of signal peptides, identified by the Inventors using proteomic analysis of *P. freudenreichii* CIRM-BIA 1 exoproteome.

**Table I**

| Protein identified | Signal peptide sequence | SEQ ID NO | |
|---|---|---|---|
| | | NT | AA |
| PF# 1058 | MSKTLSRIASVASVAALAGSITVIAGQNASA-DS Atgtcaaagacactctctcggatcgcatccgtcgcttcggttg Ccgcgctcgccggcagcatcaccgtcatcgccgggcagaa cgcgtccgccgacagc | 1 | 19 |
| PF# 1328 | MKNGLKTLLIGGVGIATLAVGGVGTAIA-DN Gtgaagaacggtctcaagaccctgctcattggtggagtcgg Catcgcgacccttgcggtcggcggcgtcggaactgccatcg cagacaat | 2 | 20 |
| PF# 1347 | MRSTTTKAFAGVAVLALALAGCGSNSGSSTKSA-DS Atgcgatccaccacgacgaaggcgtttgccggtgtcgctgtgctggcgct Ggcgcttgctggctgcggctcgaattcgggctcgtccaccaagtcggccg acagc | 3 | 21 |
| PF# 146 | MLTRKRVVAAGAAATLSLTAFAGLQPASA-AT Atgctcactcgcaagagagtggttgcagcgggagctgccgcc Accctgtccctcacggcgtttgccgggttgcagcccgccagcg ccgccacc | 4 | 22 |
| PF# 1885 | MGFRVGRRPLIGAVLAGSMATLVGCSTSGSGSGA-SS Atgggattcagggttggccgtcgtcccctcatcggggcagttctcgccgggtc Gatggcaacactcgtgggctgttccacctcgggtagcggcagtggagcctc cagc | 5 | 23 |
| PF# 190 | MQALQGRRRSRRVMAAAVAALTAMTVLPSQLNAVA-AP Atgcaggccctccaaggaaggcgccggtcacgacgggtgatggcggccgc Ggtagcagccctcaccgccatgaccgtgctgccctcccagctcaacgccgttg ctgcaccc | 6 | 24 |
| PF# 2074 | MSTGRMKFIKLAVPVIVACCLTPMAALA-DV Atgtccactggccgcatgaagttcatcaagctggcagttcctg tcatcgttgcctgctgcttgacgccaatggctgccttagctgatgtg | 7 | 25 |
| PF# 241 | MAMRARHGVVRLGLVCLTALAVFGTANVSGQVAVMA- EG Atggcgatgagggcacgtcacggcgtcgtccggcttggtctggtctgtctcaccgc attggcggtcttcggcacggcaaatgtgtcgggtcaggttgcggtgatggctgagggc | 8 | 26 |
| PF# 2732 | MNQALSTMRLKIGDSTKRIRIFFVVMAVAITLLA-GR Ttgaaccaggccctgtcgacgatgcgcctgaagatcggcgactccacc Aagcgcatccggatcttcttcgtcgtgatggccgtggcgatcaccctgctc gcgggacgg | 9 | 27 |
| PF# 279 | MRRRTTIAALAAVLSFSPLAAQA-AP Atgcgacgtcgcaccacgattgcagccctcgctg Ctgtcttgagtttcagtcccctggccgcccaggccg caccc | 10 | 28 |
| PF# 2818 | MPSHAVRETRANKLRRFLRPTVAQGVLGIAFCLVAAVGV VQI-RS Atgcctagtcatgcggtgcgggagacgcgggcgaacaagttgcgccggttcctgcggcc c Accgttgcccagggcgtgctcggtatcgcgttctgcctcgtggccgccgtcggcgtggtg ca gatccgctcc | 11 | 29 |
| PF# 2932 | MSRIQLPRLSRIAIAAAASAALIGTSFIAPATAFA-AP Atgtcacggattcaactcccccggctgagccggattgcgatcgcagc Agcagcttccgctgccctgatcggcaccagcttcatcgccccggcca cggcctttgccgcgccg | 12 | 30 |
| PF# 3042 | MKRRTLLGTLGIMGLSVPLAACS-SK Ctagccgaccttctcggccttgctggccaggtcgtc ggggatcgtgacccccatcagggaggcggccttctcatt | 13 | 31 |
| PF# 3412 | MVTGGNDMPSKRITTWPGISALSALIAGMLLAPLPVAA- DG    Ttagttgttggggacgaggagggagtggagttcgatgacgtcgagggtgggtgc Ggtggcggggcgggtgatggtttcggtgccggtgtgtccttgggcggtccaggtg atggtccaggt | 14 | 32 |
| PF# 3427 | MAMVMASLAMFGASRASA-AD Tcagccagttggtgccggccttggcgtcgg cggcgtgggatacacgcggaactgggcgcc | 15 | 33 |
| PF# 527 | MFISRFRRAAAVGLAAVTALSATACSGSSSSSSSSA-SS Atgttcatttcgcgcttccgtcgtgcggctgcggtcggcctggccgc Cgtcaccgcattgtccgccactgcctgtagcggttcctcgtcgtcgtc cagctcatccgcgagctcg | 16 | 34 |
| PF# 876 | MKSATRRPLTRWIVAFGVVLVLVIAGSVGLHASG-AL Atgaagtccgcgacgcgacgcccgctgacgcgctggattgtcgccttcg Gggtggtgttggtgctggtcatcgccgggtcggtggggctgcatgcctccg gtgccctg | 17 | 35 |
| PF# 963 | MNPFVKTARVAITSTLVAGSLATASLVFAPLAQA-DY Gtgaatcccttcgtcaagacggcgcgcgtggctatcacctcgacgc Tggtggcaggctcgctcgccactgccagcctcgtgtttgcaccactt gcacaggccgattac | 18 | 36 |

Table II below presents amino acid sequences of signal peptide, identified by the Inventors from the genomic sequence of *P. acnes,* accessible from databases (strain *P. acnes* KPA171202; accession number: NCBI: NC_006085; GenBank: AEO17283).

**Table II**

| **Protein sequence accession number** | **Signal peptide sequence** | **SEQ ID NO** | **Associated function** | **Homologous protein in *P*. *freudenreichii*** |
|---|---|---|---|---|
| PPA2239 | MSKVVASAIA | 45 | | PF1328 |
| | GALSTLSAGG | | | |
| | LTMVQA | | | |
| PPA1840 | mrkaivtpva vlavlvmalt | 46 | | PF1347 |
| | gcgqknqsgg | | | |
| PPA1786 | mastprrrwa wvlllvvasl | 47 | | PF1885 |
| | vivgvyrka | | | |
| PPA2198 | mssmkglslv latsfmlsfs | 48 | | PF2074 |
| | pgssfa | | | |
| PPA0721 | mehrygasqv sgsaprrgrg | 49 | | PF241 |
| PPA2198 | mssmkglslv latsfmlsfs | 50 | | PF3412 |
| | pgssfas | | | |
| PPA0257 | mphsdqptsk rvmsaprrrm | 51 | | PF876 |
| | pgwvpvtvgi avvvivvvav | | | |
| | ivsslrs | | | |
| AAA51650 | mfgtpsrrtf ltasalsama | 52 | hyaluronidase | |
| | laasptvtda | | | |
| | ia | | | |
| CAA67627 | mkinarfavm aasvavlmaa | 53 | triacylglycerol lipase | |
| | apiaqa | | | |
| AAT83976 | mypvhlplrn esefsfrahn | 54 | lipoprotein | |
| | hggtvpsrlt rrsvlatgav | | | |
| | alpmtaaaca | | | |
| AAT83859 | mrhmrplial slaglmtlsa | 55 | peptide-binding protein | |
| | cgedvaa | | | |
| AAT83771 | mnrtlkvaav gaiailclaa | 56 | secreted sugar-binding protein | |
| | csdpgsdsaq s | | | |
| AAT83059 | mekssfaaan mtimsepttp | 57 | secreted protease | |
| | tsqa | | | |

Advantageously, said nucleic acid sequence encoding a signal peptide can be selected from the group consisting of:
- the sequence of the signal peptide of a gene encoding a surface layer protein of a *Propionibacterium freudenreichii,* particularly of the *Propionibacterium freudenreichii* CIRM BIA 118 strain;
- the sequences having at least 80% more particularly at least 85%, at least 86%, at least 87%, at least 88%, at least 89%, at least 90%, at least 91%, at least 92%, at least 93%, at least 94%, at least 95%, at least 96%, at least 97%, at least 98% and even more particularly at least 99% of identity with said sequence over the entire length of said sequence.

Said surface layer protein can be selected from the group consisting of the surface layer protein A, the surface layer protein B, the surface layer protein C, the surface layer protein D, the surface layer protein E and the surface layer protein F of a *Propionibacterium freudenreichii,* in particular the surface layer protein A of a *Propionibacterium freudenreichii.*

As above stated, the Inventors have unexpectedly demonstrated that signal peptides of genes encoding a surface layer protein of a *Propionibacterium freudenreichii* allow the secretion into the extracellular medium of a substance of interest by a propionibacterium. These results are really surprising since surface layer proteins are, as indicated by their name, described as proteins anchored in the surface of *Propionibacterium freudenreichii* (Lortal *et al.* 1993).

More surprisingly, the Inventors have shown that said signal peptides, in particular in combination with promoters of genes encoding a surface layer protein of a *Propionibacterium freudenreichii,* allow the secretion into the extracellular medium of a substance of interest by a propionibacterium at a level up to 100 times greater than that obtained with other signal peptides in the same propionibacterium strain.

In one embodiment, said promoter and said signal peptide are the promoter and the signal peptide of the same gene encoding a surface layer protein of a *Propionibacterium freudenreichii.*

In particular, said nucleic acid sequence encoding a signal peptide is selected from the group consisting of:
- the nucleic acid sequences encoding the signal peptides of sequences SEQ ID NO: 59 to 68, in particular of sequence SEQ ID NO: 59;
- the sequences having at least 80%, more particularly at least 85%, at least 86%, at least 87%, at least 88%, at least 89%, at least 90%, at least 91%, at least 92%, at least 93%, at least 94%, at least 95%, at least 96%, at least 97%, at least 98% and even more particularly at least 99% of identity with one of said sequences over the entire length of said sequence and particularly corresponding to nucleic acid sequences encoding a signal peptide allowing the secretion, in particular into the extracellular medium, of said substance of interest by a propionibacterium.

Unexpectedly, the inventors have demonstrated that the signal peptide having the sequence set forth in SEQ ID NO: 59 in combination with the promoter of the gene encoding the Surface layer protein A of a *Propionibacterium freudenreichii* allows the secretion into the extracellular medium of a substance of interest by different strains of *Propionibacterium freudenreichii* at a level up to 100 times greater than that obtained with other signal peptides and promoters in the same *Propionibacterium freudenreichii* strain.

**Table III - Sequences of the different signal peptides corresponding to surface layer (slp) genes identified in different strains of Propionibacterium freudenreichii**

| **Strain (CIRM BIA N°)** | **Gene name** | **Locus Tag** | **Product** | **Signal peptide** | **SEQ ID NO:** |
|---|---|---|---|---|---|
| 118 | slpA | PFCIR M118_ 06465 | S-layer protein A | MATGAAAAMFVTTFAG MAPANA | 59 |
| 118 | slpB | PFCIR M118_ 02660 | S-layer protein B | MSVRKSLTGMALGLALTI TPLAGAVPASA | 60 |
| 118 | slpD | PFCIR M118_ 04435 | S-layer protein D | MRRFFSAAIAILLAATLTP ALNAPMASA | 61 |
| 121 | slpD | PFCIR M121_ 00945 | S-layer protein D | MRRFFSAAIAILLAATLTP ALNAPMASA | 62 |
| 122 | slpB | PFCIR M122_ 04010 | S-layer protein B | MSVRKSLTGMALGLALTI TPLAGAVPAAA | 63 |
| 122 | slpD | PFCIR M122_ 11375 | S-layer protein D | MRRFFSAAIAILLAATLTP ALNAPMASA | 64 |
| 122 | slpE | PFCIR M122_ 10825 | S-layer protein E | MKTRVRSRKPAAGLAGIA LFASGLSLMSTVASR | 65 |
| 125 | slpA | PFCIR M125_ 05685 | S-layer protein A | MATGAAAAMFVTTFAG MAPANA | 66 |
| 129 | slpA | PFCIR M129_ 13370 | S-layer protein A | MATGAAAAMFVTTFAG MAPANA | 67 |
| 512 | slpA | PFCIR M512_ 08945 | S-layer protein A | MATGAAAAMFVTTFAG MAPANA | 68 |

In one embodiment, the vector according to the invention comprises the nucleic acid sequence SEQ ID NO: 69 or a sequence having at least 80%, more particularly at least 85%, at least 86%, at least 87%, at least 88%, at least 89%, at least 90%, at least 91%, at least 92%, at least 93%, at least 94%, at least 95%, at least 96%, at least 97%, at least 98% and even more particularly at least 99% of identity with SEQ ID NO: 69 over the entire length of said sequence, translationally fused to said nucleic acid sequence encoding a substance of interest.

Particularly, the sequences having at least 80% of identity with the nucleic acid sequence SEQ ID NO: 69 over the entire length of said sequence, comprise a promoter and a sequence encoding a signal peptide, particularly a secretory signal peptide.

In particular, the vector according to the invention comprises the nucleic acid sequence SEQ ID NO: 69 translationally fused to said nucleic acid sequence encoding a substance of interest. The nucleic acid sequence SEQ ID NO: 69 comprises the promoter and the signal peptide of the Surface layer protein A of the *Propionibacterium freudenreichii* CIRM BIA 118.

The percentage of identity to which reference is made in the presentation of the invention are determined on the basis of global alignment of sequences to be compared, that is to say, on an alignment of sequences over their entire length, using for example the algorithm of Needlman and Wunsch 1970. This sequence comparison can be done for example using the needle software by using the parameter "Gap open" equal to 10.0, the parameter "Gap Extend" equal to 0.5, and a matrix "BLOSUM 62". Software such as needle is available on the website ebi.ac.uk worldwide, under the name "needle".

Said nucleic acid sequence encoding a signal peptide can be translationally fused with several nucleic acid sequences encoding a substance of interest. Said nucleic acid sequence encoding a signal peptide can be operatively linked to several promoters.

In the context of the present invention, said substance of interest can be any molecule of interest. Particularly said substance of interest is of eukaryotic origin, more particularly of animal origin and even more particularly of mammalian origin. In particular, said substance of interest can originate from mammals selected from the group consisting of rodents (e.g. mice, rats, rabbits, Chinese pigs, hamsters), canidae (e.g., dogs),felidae (e.g., cats), domestic livestock (e.g.cows, pigs, goats, sheeps, horses), and humans.

In one embodiment, said substance of interest is a peptide or a protein chosen in the group consisting of antibodies, receptor ligands, hormones, cytokines, growth factors, cell adhesion molecules, blood clotting factors, enzymes, fragments thereof and combinations thereof, more particularly in the group consisting of cytokines, antibodies and hormones, and even more particularly from the group consisting of proapoptotic TRAIL protein and the C-terminal extracellular domain of the TRAIL protein.

A "fragment" of a protein or peptide is a protein or a peptide of amino acid sequence of smaller length but included in the one of said initial peptide or protein. A protein "fragment" could be a peptide, for example.

In a preferred embodiment, the vector according to the present invention makes it possible to express and secrete the proapoptotic TRAIL protein (TNF-related apoptosis-inducing ligand, also called TNSF10, TL2, CD253 and Apo-2L), a cytokine of the TNF family. The sequence of the *Homo sapiens* TRAIL protein can be the sequence set forth in SEQ ID NO: 58 (accession number AAC50332.1).

Thus, in one embodiment, said substance of interest can be the proapoptotic TRAIL protein or the C-terminal extracellular domain of the TRAIL protein, in particular the *Homo sapiens* proapoptotic TRAIL protein or the C-terminal extracellular domain of the *Homo sapiens* TRAIL protein.

In particular, said substance of interest is the C-terminal extracellular domain of the TRAIL protein consisting of the sequence from amino acids 114 to 281 of the sequence set forth in SEQ ID NO: 58.

According to the literature, TRAIL is an antineoplastic agent with strong potential because it induces the death of many tumor cells, independently of p53 and Pgp180 (MDR, multidrug resistance). TRAIL also inhibits the growth of xenografted colon tumors in nude mice (Ashkenazi *et al.,* 1999). Quite interestingly, TRAIL has little cytotoxic effect on most normal tissues (Ashkenazi *et al.,* 1999), including human colon epithelium (Sträter *et al.,* 2002).

Other teams very recently expressed the active C-terminal extracellular domain of TRAIL in bacteria such as *Salmonella typhimurium* (Ganai *et al.,* 2009), *Bifidobacterium longum* (Hu *et al.,* 2009) and *E. coli* (Zhang *et al.,* 2010). In this work, salmonellas, bifidobacteria and coliform bacteria are proposed as systemic TRAIL delivery vectors in cancer models.

However, propionibacteria have major advantages compared to other bacteria such as salmonellas, bifidobacteria and coliform bacteria.

First, dairy propionibacteria enable local delivery because they target colon epithelial cells and have an active fermentative metabolism in the human colon (Hervé *et al.,* 2007), which enables site-specific delivery of TRAIL.

Second, dairy propionibacteria themselves have proapoptotic properties. It was recently shown *in vitro* that dairy propionibacteria, by the SCFA resulting from their fermentative metabolism, induce the apoptosis of two human colon adenocarcinoma cell lines (Caco2 and HT29) (Jan *et al.,* 2002). This effect is directly related to the release of propionate by these bacteria and to their action on cancer cell mitochondria. It has also been shown that at extracellular pH (pHₑ) between 6 and 7.5, SCFA (propionate and acetate SCFA) induce apoptotic death whereas at pHₑ=5.5, they induce necrotic death in HT29 human colon cancer cells (Lan *et al.,* 2007a). *In vivo,* these food-quality (GRAS: generally regarded as safe) bacteria adapt and survive in the digestive tract of animals and humans with an efficiency that, although strain-dependent, exceeds that of other probiotics (Hervé *et al.,* 2007). Moreover, they express in the intestine enzymatic activities characteristic of their fermentative metabolism by producing an increase in SCFA concentrations (Lan et al., 2007b) and induce an increase in apoptosis in the mucosa of the colon of rats treated with 1,2-dimethylhydrazine (Lan *et al.,* 2008). The Inventors have further shown a synergistic action with *TRAIL in vitro* as illustrated in the examples below.

In one embodiment, the vector according to the invention comprises:
- at least one promoter operatively linked to
- at least one nucleic acid sequence encoding a signal peptide translationally fused to
- at least one nucleic acid sequence encoding a substance of interest;

■ wherein said nucleic acid sequence encoding a signal peptide is selected from the group consisting of:
   - the sequence of the signal peptide of a gene encoding a surface layer protein of a *Propionibacterium freudenreichii;* and
   - the sequences having at least 80%, more particularly at least 85%, at least 86%, at least 87%, at least 88%, at least 89%, at least 90%, at least 91%, at least 92%, at least 93%, at least 94%, at least 95%, at least 96%, at least 97%, at least 98% and even more particularly at least 99% of identity with said sequence over the entire length of said sequence;
   and
■ wherein the sequence of said promoter is selected from the group consisting of:
   - the sequence of the promoter of a gene encoding a surface layer protein of a *Propionibacterium freudenreichii;* and
   - the sequences having at least 80%, more particularly at least 85%, at least 86%, at least 87%, at least 88%, at least 89%, at least 90%, at least 91%, at least 92%, at least 93%, at least 94%, at least 95%, at least 96%, at least 97%, at least 98% and even more particularly at least 99% of identity with said sequence over the entire length of said sequence.

A particularly preferred vector according to the invention is the vector pCHH04: TRAIL comprising:
- the promoter of the Surface layer protein A of the *Propionibacterium freudenreichii* CIRM BIA 118, operatively linked to
- a nucleic acid sequence encoding the signal peptide of the Surface layer protein A of the *Propionibacterium freudenreichii* CIRM BIA 118, translationally fused to
- a nucleic acid sequence encoding the sequence from amino acids 114 to 281 of the TRAIL protein sequence set forth in SEQ ID NO: 58.

A *P. freudenreichii* CIRM-BIA-118 comprising said vector pCHH04: TRAIL has been deposited on November 13, 2012 under number CNCM I-4692 with the *Collection Nationale de Cultures de Microorganismes* (CNCM, Institut Pasteur, 25 rue du Docteur Roux, 75724 Paris Cedex 15, France).

Thus, a particularly preferred vector according to the invention is the vector comprised in the propionibacterium deposited on November 13, 2012 under number CNCM I-4692 with the *Collection Nationale de Cultures de Microorganismes* (CNCM, Institut Pasteur, 25 rue du Docteur Roux, 75724 Paris Cedex 15, France).

The present invention further relates to a bacterium comprising at least one vector according to the invention as previously defined.

Said bacterium comprising a vector according to the invention, can belong to the order *Actinomycetales,* in particular to the genus *Propionibacterium,* and more particularly to a species selected from the group consisting of *Propionibacterium freudenreichii, Propionibacterium jensenii, Propionibacterium thoenii, Propionibacterium acidipropionicii, Propionibacterium acnes, Propionibacterium granulosum, Propionibacterium avidum, Propionibacterium propionicum* and *Propionibacterium microaerophilum* and even more particularly the species *Propionibacterium freudenreichii.*

In particular, said bacterium comprising a vector according to the invention can be selected from the group consisting of *Propionibacterium freudenreichii freudenreichii* and *Propionibacterium freudenreichii shermanii.*

Advantageously, the propionibacterium comprising at least one vector according to the invention can be selected from the group consisting of CIRM BIA 512 strain, CIRM BIA 125 strain, CIRM BIA 118 strain, CIRM BIA 129 strain, CIRM BIA 138 and CIRM BIA 122 strain, particularly CIRM BIA 512 strain, CIRM BIA 125 strain, CIRM BIA 118 strain, CIRM BIA 129 strain and CIRM BIA 122 strain, more particularly CIRM BIA 512 strain, CIRM BIA 125 strain, CIRM BIA 118 strain and CIRM BIA 129 strain, even more particularly CIRM BIA 512 strain, CIRM BIA 125 strain, CIRM BIA 118 strain and even more particularly CIRM BIA 512 strain and CIRM BIA 118 strain and even more particularly CIRM BIA 118 strain.

In one embodiment, the propionibacterium comprising at least one vector according to the invention is a CIRM BIA 138 strain. This strain also named TL 133 strain in the article of Lan *et al.* (2007b) has the advantage to be particularly tolerant towards digestive stresses.

Advantageously, the vector can be carried by the chromosome of the bacterium according to the invention. The vector can be integrated, for example, in the chromosome of the host cell by homologous recombination as described by Deutsch *et al.* (2012).

One particularly preferred bacterium according to the invention is the propionibacterium deposited on November 13, 2012 under number CNCM I-4692 with the *Collection Nationale de Cultures de Microorganismes* (CNCM, Institut Pasteur, 25 rue du Docteur Roux, 75724 Paris Cedex 15, France).

If the substance of interest to be secreted lends itself to such a use (an anorexiant peptide, for example), the bacterium according to the invention could be used as a probiotic food or dietary supplement for mammals, in particular humans. Advantageously, the bacterium can be integrated in the mammal's food in the form of a fermented dairy product (e.g., fermented milk, fermented whey, cheese).

The present invention further relates to a composition comprising at least one vector according to the invention and/or at least one bacterium according to the invention.

Said composition according to the invention can be a dietary or a pharmaceutical composition.

The term "dietary compositions" comprises any type of food/feed and beverages, also including clinical nutrition and dietary supplements. The dietary compositions according to the present invention may further contain protecting components (such as carbohydrates, betaines, fibers, polyols), protective hydrocolloids (such as gums, proteins, modified starches), binders, film-forming agents, encapsulating agents/materials, wall/shell materials, matrix compounds, coatings, emulsifiers, surface active agents, solubilising agents (oils, fats, waxes, lecithins etc.), adsorbents, carriers, fillers, co-compounds, dispersing agents, wetting agents, processing aids (solvents), flowing agents, taste-masking agents, weighting agents, jellyfying agents, gel-forming agents, antioxidants and antimicrobials.

Said pharmaceutical composition according to the invention can comprise an effective amount of at least one vector according to the invention and/or at least one bacterium according to the invention and at least one pharmaceutically acceptable carrier (or excipient).

Such therapeutically effective amount can be determined by one skilled in the art by routine tests including assessment of the effect of administration of said components (vector according to the invention and/or bacterium according to the invention) on the pathologies and/or disorders which are sought to be prevent and/or to be treated by the administration of said drug (or pharmaceutical composition).

For example, such tests can be implemented by analyzing both quantitative and qualitative effect of the administration of different amounts of said aforementioned components (vector according to the invention and/or bacterium according to the invention) on a set of markers (biological and/or clinical) characteristics of said pathologies and/or of said disorders, in particular from a biological sample of a subject.

The present invention further relates to a vector according to the invention or a bacterium according to the invention for use as a medicament.

The invention further relates to a vector according to the invention or a bacterium according to the invention for use as a medicament in the prevention and/or treatment of at least one disease selected from the group consisting of allergies, hypertension (e.g., the substance of interest has a hypotensive activity), obesity (e.g., the substance of interest has an anorexiant activity), cancers, in particular colorectal cancers (e.g., the substance of interest has a proapoptotic activity) and inflammatory colon diseases, in particular Crohn's disease (wherein the substance of interest is advantageously an anti-inflammatory cytokine, for example IL-10).

The invention further relates to a vector according to the invention or a bacterium according to the invention for use as a medicament in the prevention of at least one microbial infection, including a viral, bacterial, fungal or parasitic infection. In this case, the substance of interest can be an antigen or an epitope. Thus, said medicament can be a vaccine, which can further comprise an immune adjuvant.

The invention further relates to a vector according to the invention or a bacterium according to the invention, in particular a bacterium belonging to the genus *Propionibacterium* and more particularly to the species *Propionibacterium freudenreichii* for use as a medicament in the prevention and/or treatment of cancer; in particular wherein said substance of interest is an anti-tumoral agent like the proapoptotic TRAIL protein or the C-terminal extracellular domain of the TRAIL protein (in particular of sequence from amino acids 114 to 281 of the protein TRAIL sequence set forth in SEQ ID NO: 58).

As used herein the term "anti-tumoral agent" relates to any compound having an anti-tumoral effect. The anti-tumoral agent can have a preventing or inhibiting effect on the formation or growth of tumors or tumor cells. This can be a pro-apoptotic, an anti-proliferative, a cytostatic or a cytotoxic effect.

The invention further relates to the use of a vector or a bacterium according to the invention for the preparation of a medicament for the prevention and/or treatment of at least one disease, in particular selected from the group consisting of allergies, hypertension (e.g., the substance of interest has a hypotensive activity), obesity (e.g., the substance of interest has an anorexiant activity), cancers, in particular colorectal cancers (e.g., the substance of interest has a proapoptotic activity) and inflammatory colon diseases, in particular Crohn's disease (wherein the substance of interest is advantageously an anti-inflammatory cytokine, for example IL-10).

The invention further relates to the use of a vector or a bacterium according to the invention for the preparation of a medicament in the prevention of at least one microbial infection, including a viral, bacterial, fungal or parasitic infection. In this case, the substance of interest can be an antigen or an epitope. Thus, said medicament can be a vaccine, which can further comprise an immune adjuvant.

The medicament or the composition according to the invention can be manufactured in a conventional way. The medicament (pharmaceutical composition) in accordance with the invention can moreover include one or more pharmaceutically acceptable excipients or additives such as diluents, adjuvants, anti-foaming agents, stabilizers, dispersants, colorants, preservatives, etc. Inert excipients or adjuvants can be used in such a way that, in the drugs according to the present invention, the only therapeutic agents will be the vector and/or the bacterium. Nevertheless, the medicament (pharmaceutical composition) according to the present invention can include one or more other therapeutically or prophylactically active agents, in addition to the vector and/or the bacterium. Advantageously, the combination of several therapeutic agents, including at least the vector and/or the bacterium, will have a better therapeutic or prophylactic action than when the vector and/or the bacterium are the only therapeutic agents present in the medicament (pharmaceutical composition). This better action can be, among others:
- a better dose-effect relationship;
- a therapeutic or prophylactic effect that is more stable or longer lasting over time;
- a better administration of the medicament (pharmaceutical composition);
- a synergy of action between at least two therapeutic agents present in the medicament (pharmaceutical composition).

The various means of the present invention (vector, bacterium, medicament, pharmaceutical composition) as described above are preferably administered to a mammal for the secretion of the substance of interest in the small intestine and/or the colon, preferably the colon, of said mammal.

The term "mammal" is defined in its usual sense. Examples of mammals include bovines; pigs; goats; sheep; horses; rodents such as mice, rabbits, rats and hamsters; felines and canines, including domestic animals such as cats and dogs. A preferred mammal in the context of the invention is a human.

The means of the invention (vector, bacterium, medicament, pharmaceutical composition) can be administered by any suitable conventional route, in particular selected from the oral, subcutaneous, intramuscular, intravenous, intrarectal, enema and intratracheal routes. Oral administration is preferred, wherein the drug is in the form of tablets, hard gelatin capsules (e.g., gastroprotective gelatin capsules), soft capsules, powders for direct use or for dilution (e.g., lyophilisates), syrups, gels, etc. Said means can be administered in a single or repeated dose one or more times spaced over a certain interval of time. The suitable administration route and dosing schedule can vary according to various parameters, such as the subject to be treated and/or the substance of interest.

The invention also relates to a method for therapeutic or prophylactic treatment of a subj ect in need thereof, comprising the step of administering to said subject a therapeutically effective amount of at least one compound selected from the group consisting of:
- a vector according to the invention;
- a bacterium according to the invention; and
- a medicament (pharmaceutical composition) according to the invention.

The invention also relates to a method for therapeutic or prophylactic treatment of a disease, in particular a cancer, more particularly a colorectal cancer, comprising the step of administering to a subject in need thereof a therapeutically effective amount of at least one compound selected from the group consisting of:
- a vector according to the invention;
- a bacterium according to the invention, in particular a bacterium belonging to the genus *Propionibacterium* and more particularly to the species *Propionibacterium freudenreichii;* and
- a medicament (pharmaceutical composition) according to the invention;
particularly wherein said substance of interest is the proapoptotic TRAIL protein or the C-terminal extracellular domain of the TRAIL protein (in particular of sequence from amino acids 114 to 281 of the protein TRAIL sequence set forth in SEQ ID NO: 58).

The present invention further relates to the use of a vector and/or a bacterium according to the invention to produce and secrete, preferably into the extracellular medium, one or more substance of interest.

The present invention further relates to a method for producing and secreting into the extracellular medium, at least one substance of interest, by at least one bacterium according to the invention, said method comprising the steps of:
- culturing said bacterium under suitable conditions; and
- recovering the culture medium containing said substance of interest; and
- optionally, purifying said substance of interest.

Preferably, the method according to the invention makes it possible to produce and secrete substances of interest on a large scale, that is, on an industrial scale.

The "suitable conditions" (in terms of the composition of the culture medium, temperature, time, ventilation, stirring, etc.) for culturing bacteria and in particular propionibacteria are known to those persons skilled in the art (see in particular documents US 20090312425 in the name of Meiji Dairies Corp. and CN 101045910 in the name of Nanjing University of Technology). Once again, propionibacteria are robust, are able to grow on particular substrates such as whey or molasses and are able to adapt to non-standard culture conditions, characteristics which a large number of other bacteria do not share.

Purification of substance of interest calls upon the general knowledge of those persons skilled in the art and can be carried out without any difficulty using classic techniques.

The invention further relates to a product comprising:
- at least one vector according to the invention and/or at least one bacterium according to the invention; and
- at least another active agent, in particular chosen in the group consisting of an anti-tumoral agent, an anti-inflammatory agent and an immunomodulatory agent, more particularly an anti-tumoral agent;
as a combination product for simultaneous, separate or sequential use for the prevention and/or treatment of at least one disease, in particular selected from the group consisting of allergies, hypertension (e.g., the substance of interest has a hypotensive activity), obesity (e.g., the substance of interest has an anorexiant activity), cancers, in particular colorectal cancers (e.g., the substance of interest has a proapoptotic activity) and inflammatory colon diseases, in particular Crohn's disease (wherein the substance of interest is advantageously an anti-inflammatory cytokine, for example IL-10).

Said active agent can be a short-chain fatty acid (SCFA), preferably propionate or acetate.

The invention also relates to an isolated nucleic acid molecule of sequence selected from the group consisting of:
- the nucleic acid sequences encoding the peptides of sequences SEQ ID NO: 59 to 68;
- the nucleic acid sequence SEQ ID NO: 69; and
- the sequences having at least 80%, more particularly at least 85%, at least 86%, at least 87%, at least 88%, at least 89%, at least 90%, at least 91%, at least 92%, at least 93%, at least 94%, at least 95%, at least 96%, at least 97%, at least 98% and even more particularly at least 99% of identity with one of said sequences over the entire length of said sequence.

Particularly, the sequences having at least 80% of identity with one of the nucleic acid sequences encoding the peptides of sequences SEQ ID NO: 59 to 68 over the entire length of said sequence, encode a signal peptide, particularly a secretory signal peptide.

Particularly, the sequences having at least 80% of identity with the nucleic acid sequence SEQ ID NO: 69 over the entire length of said sequence, comprise a promoter and a sequence encoding a signal peptide, particularly a secretory signal peptide.

The invention further relates to a vector comprising at least one nucleic acid sequence selected from the group consisting of:
- the nucleic acid sequences encoding the peptides of sequences SEQ ID NO: 59 to 68;
- the nucleic acid sequence SEQ ID NO: 69; and
- the sequences having at least 80%, more particularly at least 85%, at least 86%, at least 87%, at least 88%, at least 89%, at least 90%, at least 91%, at least 92%, at least 93%, at least 94%, at least 95%, at least 96%, at least 97%, at least 98% and even more particularly at least 99% of identity with one of said sequences over the entire length of said sequence.

The invention also relates to a bacterium comprising a vector comprising at least one nucleic acid sequence selected from the group consisting of:
- the nucleic acid sequences encoding the peptides of sequences SEQ ID NO: 59 to 68;
- the nucleic acid sequence SEQ ID NO: 69; and
- the sequences having at least 80%, more particularly at least 85%, at least 86%, at least 87%, at least 88%, at least 89%, at least 90%, at least 91%, at least 92%, at least 93%, at least 94%, at least 95%, at least 96%, at least 97%, at least 98% and even more particularly at least 99% of identity with one of said sequences over the entire length of said sequence.

The advantageous embodiments are as describe above.

The present invention is illustrated by the following figures:

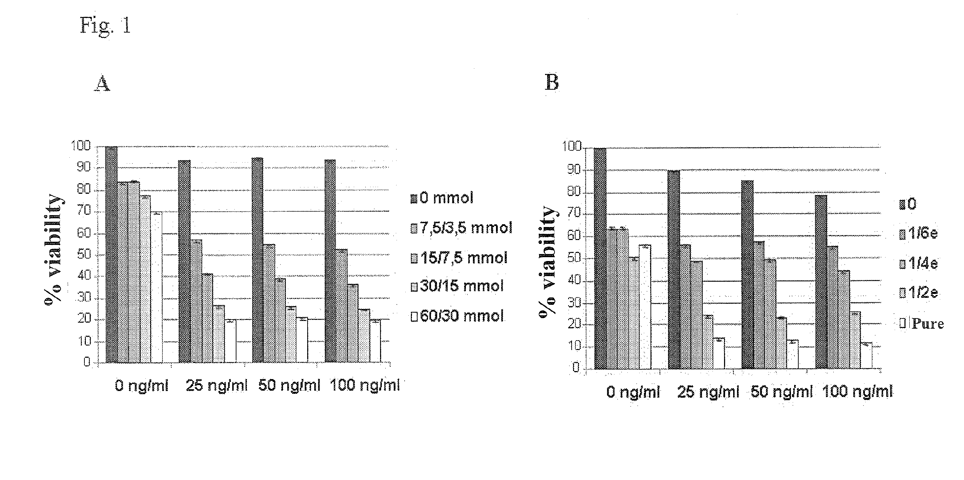

Figure 1: Graphs illustrating the synergy observed *in vitro* between TRAIL and *P. freudenreichii* metabolites. HT29 colon cancer cells were treated with sublethal doses of TRAIL (25 ng/ml, 50 ng/ml and 100 ng/ml). Various doses of SCFA (propionate/acetate SCFA, figure 1A) or of *P. freudenreichii* supernatant (figure 1B) were used in co-treatment. Viability of the HT29 cells was determined after 24 hours of treatment.

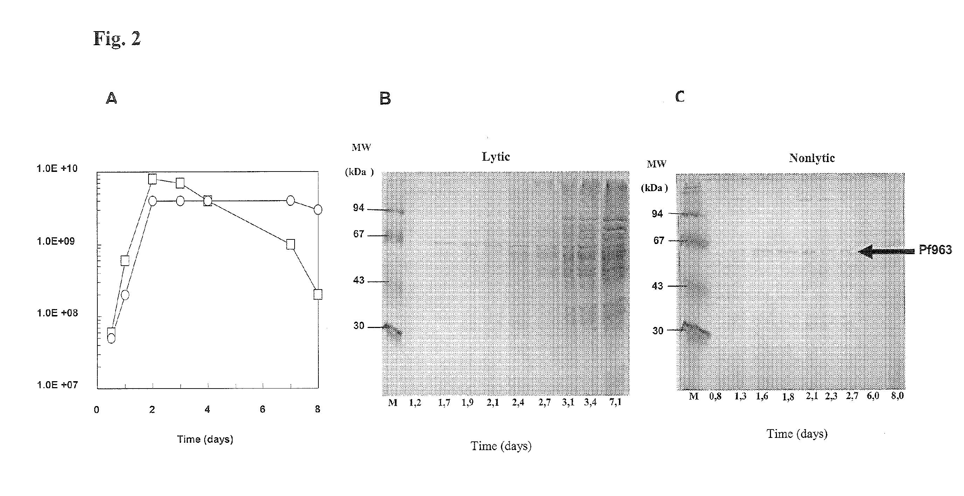

Figure 2: Identification of the protein secreted in the majority by *P*. *freudenreichii,* PF963. A: growth of two strains of *P.freudenreichii,* one autolytic ( 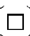 ) and the other nonlytic (o), B and C: electrophoretic analysis (SDS-PAGE) of proteins secreted by a strain of lytic (B) and nonlytic (C) *P. freudenreichii.* Protein PF963 was identified by mass spectrometry.

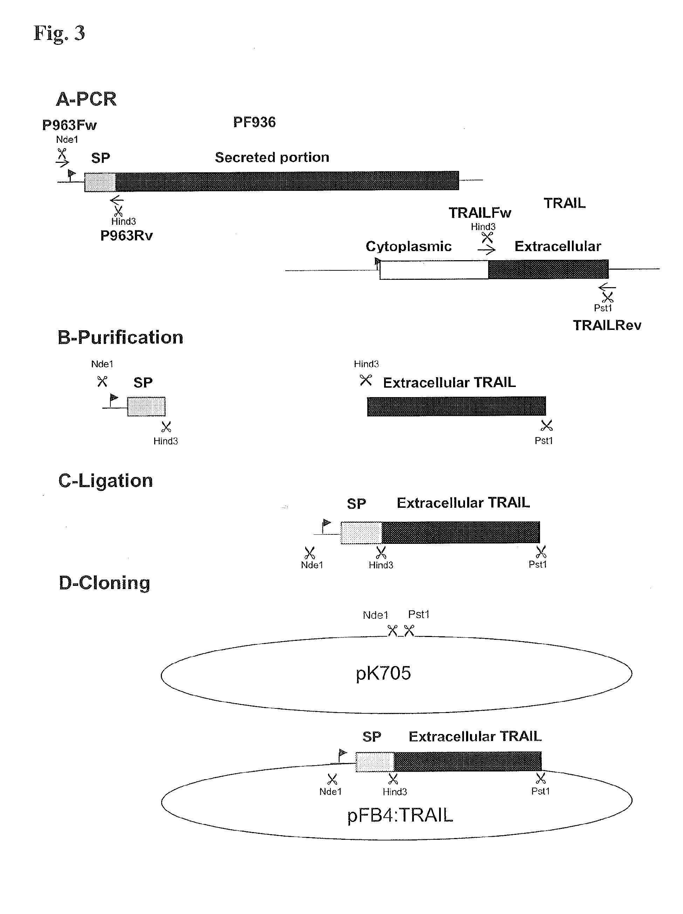

Figure 3: Diagram detailing the cloning strategy to obtain the pFB4:TRAIL plasmid (deposited with the CNCM on July 23, 2009 under number I-4213). A: the promoter region and signal peptide of the *P.freudenreichii* protein PF963 were amplified by PCR with introduction (PCR mutagenesis) of restriction sites *Nde*1 and *Hin*dIII*.* The active extracellular portion of TRAIL (residues 114 to 281) was amplified by PCR with introduction of restriction sites *Hin*dIII and *Pst*1*.* B and C: the two PCR products were purified and linked in order to obtain the ligation product SP-TRAIL. D: plasmid pK705 was opened by digestion using two enzymes *Nde*1 and *Pst*1*.* The ligation product SP-TRAIL was introduced into the open plasmid. The new plasmid pFB4 includes a promoter and a signal peptide enabling the secretion, in particular into the extracellular medium, by *P*. *freudenreichii* of a heterologous protein. The arrows and the scissors represent PCR primers and restriction sites, respectively.

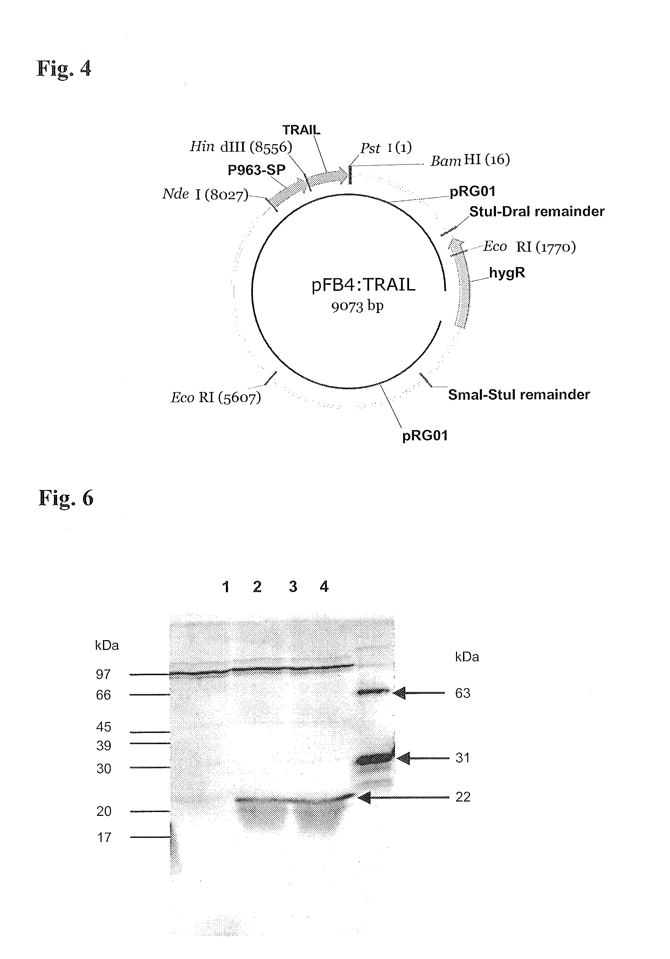

Figure 4: Map of the pFB4:TRAIL plasmid (deposited with the CNCM on August 13, 2009 under number I-4213).

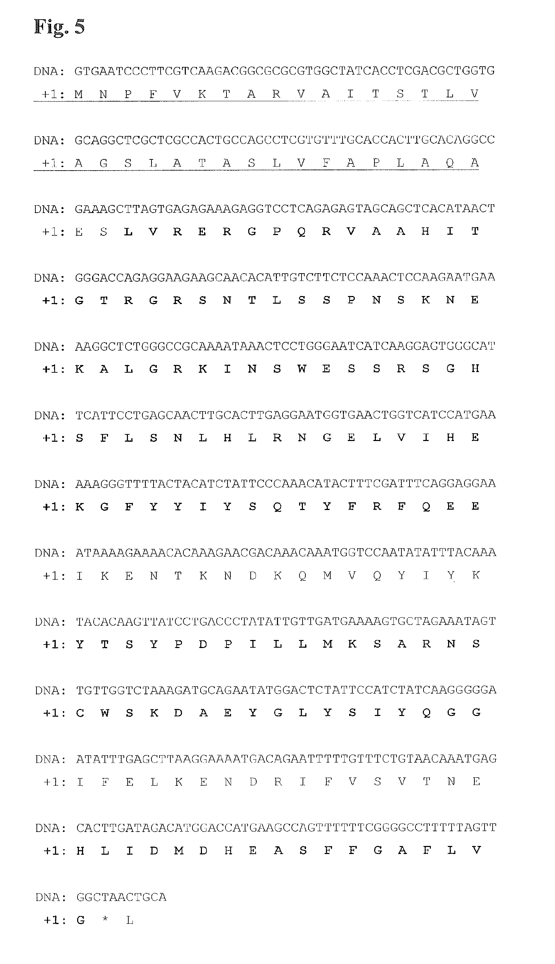

Figure 5: Sequence of the fusion protein coded for by the pFB4:TRAIL plasmid. In this sequence, the underlined region corresponds to the PF963 protein signal sequence and the region in bold corresponds to the TRAIL extracellular domain sequence (residues 114 to 281).
Figure 6: Detection by Western blot of the fusion protein coded for by the pFB4:TRAIL plasmid. The samples deposited were culture supernatants of wild *P. freudenreichii* CIP103027 (1) or of *P. freudenreichii* CIP103027 carrying the pFB4:TRAIL plasmid (2 and 3). A solution of Super*Killer*TRAIL™ (Alexis Biochemicals, Coger, France) was deposited as positive control (4). The Western blot was developed using a commercial "PAb to TRAIL" antibody (Alexis Biochemicals).

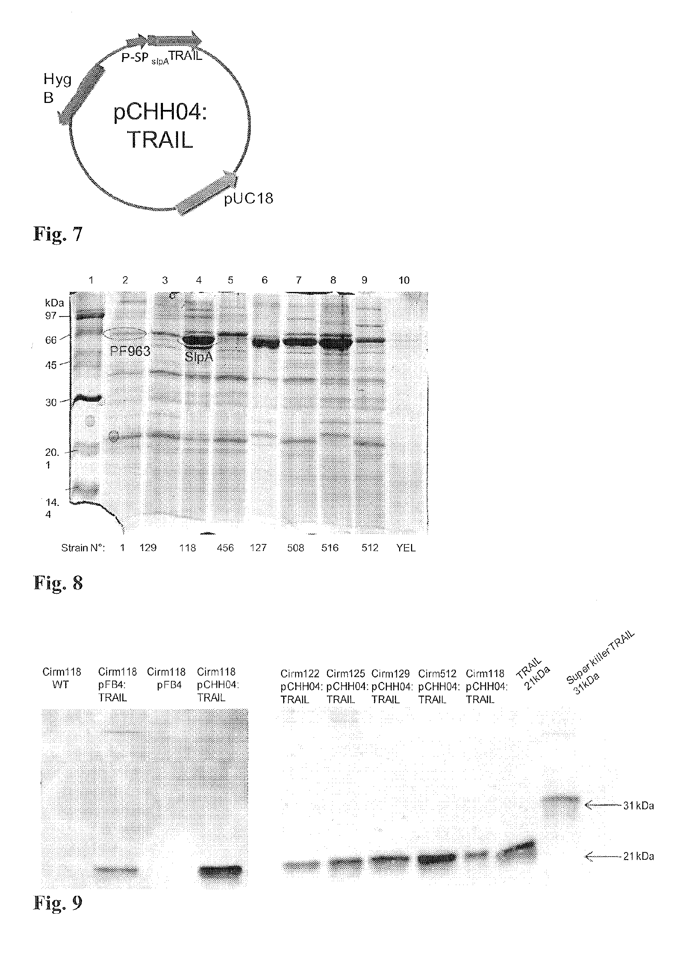

Figure 7: Map of the pCHH04: TRAIL plasmid (deposited with the CNCM on November 13, 2012 under number CNCM I-4692).
Figure 8: Proteomic comparative analysis of *Propionibacterium freudenreichii* exoproteomes. Strains CIRM BIA 1, 129, 118, 456, 127, 508, 516 and 512 were grown in YEL medium and the culture supernatants analyzed by SDS PAGE followed by Coomassie Blue staining. The reference strain CIRM BIA 1, the first sequenced in 2010, secretes 4 proteins with equivalent efficiency, including PF963. Other strains, sequenced since then (unpublished data), secrete a major extracellular 60 kDa protein abundantly. This was identified in strain 118 as SlpA.
Figure 9: Western Blot detection. The plasmids pFB4:TRAIL and pCHH04:TRAIL are compared within the same CIRM BIA 118 strain with respect to TRAIL secretion into the extracellular medium. Secretion of TRAIL into the extracellular medium is then compared in different strains harboring the pCHH04:TRAIL plasmid :The pCHH04:TRAIL plasmid was transformed into different strains of *Propionibacterium freudenreichii* and the resulting supernatants were analyzed with respect to TRAIL secretion by western blotting.

The present invention will be explained in detail with examples in the following, but the technical scope of the present invention is not limited to these examples.

### EXAMPLES

### I-Induction of the intrinsic mitochondrial pathway of apoptosis by Propionibacterium freudenreichii.

During preliminary studies, the Inventors showed that certain selected strains of *P. freudenreichii* surviving the stresses undergone during intestinal transit in humans (Hervé *et al.,* 2007), as well as in the rat (Lan *et al.,* 2007b), express the genes coding for fermentative metabolism enzymes and produce propionate and acetate short-chain fatty acids (SCFA) *in situ* in the colon (Lan *et al.,* 2007b)*.*

Furthermore, this bacterium induced the apoptosis of human colon adenocarcinoma cells *in vitro* via these SCFA which act on cancer cell mitochondria (Jan *et al.,* 2002). The mitochondrial pathway of apoptosis induction has been clearly identified in the triggering of programmed cell death of HT29 cells by dairy propionibacteria (Jan *et al.,* 2002; Lan *et al.,* 2007a). Said SCFA cause the opening of mitochondrial permeability transition pores (PTP), the depolarization of mitochondria, the leaking of proapoptotic mitochondrial proteins and the activation of effector caspases.

Such an induction of apoptosis was then researched *in vivo* in a rat model of human digestive flora. Rats were treated or not treated by the carcinogen dimethylhydrazine (DMH) for the purpose of causing the appearance of damaged colonic epithelial cells likely to develop into colon cancer. These rats received by gavage, or did not receive, the *P.freudenreichii* bacterium. Colonic epithelial cell apoptosis and proliferation were quantified by anatomopathological analysis of histological sections of colon. The administration in healthy rats of *P*. *freudenreichii* had no effect on these parameters. On the other hand, a significant increase in apoptosis was observed in the rats treated with DMH (Lan *et al.,* 2008). It thus appears that a specific apoptosis of cancer cells can be induced by dairy propionibacteria.

### II-Induction of the extrinsic pathway of apoptosis by TRAIL via death receptors, synergy with Propionibacterium freudenreichii.

TRAIL is a cytokine capable of inducing the apoptosis of human colon cancer cells by binding to death receptors. TRAIL thus induces a different apoptotic pathway on the cellular and molecular levels and potentiates the action of other proapoptotic molecules used in cancer chemotherapy (Lacour *et al.,* 2001; Lacour *et al.,* 2003; Meurette *et al.,* 2005; Meurette *et al.,* 2006). Cell death induced by TRAIL or by SCFA is promoted by an acidic environment (Meurette *et al.,* 2007; Lan *et al.,* 2007a).

By viability tests (figure 1), and by *in vitro* apoptosis quantification methods (Hoechst staining and caspases activity, data not shown), the Inventors showed a synergistic proapoptotic effect of the cytokine TRAIL in combination with the propionate/acetate mixture or the propionibacteria culture supernatant in HT29 human colon cancer cells (figure 1). More precisely, the viability illustrated in figure 1 was determined using the following cytotoxicity test. HT29 human colon cancer cells (ATCC, Biovalley) were cultured in 96-well plates (30,000 cells/well) for 24 hours. They were then treated with TRAIL (0 ng/ml, 25 ng/ml, 50 ng/ml and 100 ng/ml) (Super*Killer*TRAIL™, Alexis Biochemicals, Coger, France) in the presence of increasing concentrations of propionate/acetate (7.5 mmol/3.5 mmol; 15 mmol/7.5 mmol; 30 mmol/15 mmol; 60 mmol/30 mmol) or of bacterial supernatant (*P.freudenreichii* bacteria) (1/6, 1/4, 1/2, pure). At the end of treatment (24 hours), the medium was discarded and the adherent cells were washed three times with 1X PBS (100 µl/well) and fixed in 99% ethanol (100 µl/well) for 30 minutes. After discarding the ethanol, the fixed cells were air dried and then stained for 30 minutes with methylene blue (diluted in 1X borate buffer). After three washings in water and drying (roughly 30 minutes), 100 µl of hydrochloric acid (0.1 N) was added to the wells. The plates were then analyzed by spectrophotometer at a wavelength of 620 nm (iEMS Reader MF; Labsystems, Helsinki, Finland).

Figure 1 shows that sublethal doses of TRAIL (25 ng/ml, 50 ng/ml and 100 ng/ml) do not significantly induce cell death during the treatment period. Moreover, the smallest doses of SCFA alone induce little or no cell death, but induce massive death in the presence of TRAIL (figures 1A and 1B). These results show a synergy of proapoptotic action on human colon cancer cells between SCFA metabolites produced by *P.freudenreichii* and TRAIL.

### III-Development of a first recombinant propionibacterium with the goal of inducing both the intrinsic and extrinsic apoptotic pathways.

### III.1 Summary

The Inventors sought to make a bacterium, harmless to healthy cells, produce inducers of the two apoptotic pathways. These inducers are the SCFA produced by *P. freudenreichii* for the intrinsic pathway and TRAIL for the extrinsic pathway. Since propionibacteria have a positive tropism for the mucosa of the colon, said recombinant bacterium will not only be likely to produce TRAIL *in situ* in the colon, but also to carry SCFA and TRAIL toward colon epithelial cells.

To this end, a recombinant propionibacterium expressing TRAIL fused with a secretion signal peptide was developed for *in situ* production in experimental models of cancer colon.

Briefly, the major protein secreted by *P. freudenreichii* during its growth and in the absence of lysis, named PF963, was identified. The experimental procedure (e.g., electrophoresis, trypsinolysis, nano-LC and MS/MS) which led to the identification of PF963 is similar to that which had previously enabled the Inventors to identify GAPDH (Tarze *et al.,* 2007). Very briefly, the supernatant of the nonlytic strain of *P. freudenreichii* was analyzed by electrophoresis. The gel fragment containing the major protein secreted was removed, rinsed and then subjected to "in gel" trypsin proteolysis. The resulting peptides were separated by nano-LC and then analyzed with tandem mass spectrometry (MS/MS).

PF963 is an enzyme secreted via the machinery of the "Sec" pathway which recognizes and cleaves a signal peptide (SP). By genetic engineering, said SP was fused with the active C-terminal extracellular domain of TRAIL. This construction was carried out in *E. coli* on a cloning plasmid. The fusion thus obtained was introduced into an expression vector (pK705) previously developed for the cloning and expression of propionibacterial genes in dairy propionibacteria and efficient in *P. freudenreichii* (Kiatpapan *et al.,* 2000) in order to express the fusion protein. The expression and the extracellular addressing of the fusion protein were then analyzed by Western blot.

According to figure 2A, the growth of *P*. *freudenreichii* shows that certain strains are lysed (□) and others not (o). In the latter case, protein PF963 is secreted in the medium (figure 2C) without leakage of cytoplasmic proteins as in the case of spontaneous bacterial lysis (figure 2B). The upstream portion of the PF963 gene, comprising the promoter and the signal peptide, was amplified by PCR and fused with the C-terminal portion of TRAIL (figures 3A to 3C). The following step consisted of its introduction in a *P. freudenreichii* expression plasmid (figure 3D).

### III.2 Obtaining the strain Propionibacterium freudenreichii subsp. shermanii CIRM BIA 1 carrying the pFB4:TRAIL plasmid.

### III.2.1 Identification of the protein PF963 secreted by Propionibacterium freudenreichii subsp. shermanii.

In order to identify a secreted protein, strains were screened on the basis of aptitude for autolysis. Indeed, it is known that certain strains of said bacterium make use of a programmed cell suicide, autolysis. In this case, cytoplasmic proteins are released in the surrounding medium. On the other hand, other strains, including strain CIRM BIA 1, do not undergo autolysis and on the contrary make use of a tolerance reaction with respect to various stresses, called the starvation-induced multi-tolerance response. In principle, these nonlytic strains thus only release actively secreted proteins and do not release proteins by accident. Figure 2A shows the evolution of the bacterial population for an autolytic strain (□) and for a nonlytic strain (o) of *Propionibacterium freudenreichii* subsp. *shermanii.* Figure 2C shows the electrophoretic analysis (SDS-PAGE) of proteins secreted by a nonlytic strain, CIRM BIA 1. This analysis reveals several secreted proteins, including protein PF963, identified in the culture supernatant of all the nonlytic strains tested. This protein was cut out of a preparative SDS-PAGE gel and subjected to digestion by trypsin. The resulting peptides were analyzed by electrospray ionization tandem mass spectrometry (ESI-MS/MS) on a hybrid triple quadrupole time-of-flight apparatus (QSTAR®XL, Applied Biosystems) according to a standard laboratory procedure (Science and Technology of Milk and Eggs) described in Tarze *et al.* (2007).

By this analysis, the Inventors identified protein PF963, a secreted bacterial wall peptidase belonging to the NlpC/P60 family. The complete sequence of protein PF963 (SEQ ID NO 36; table I) can be deduced after determination of the complete sequence of the genome of strain CIP103027 by the Inventors.

III.2.2 Fusion of the N-terminal portion of protein PF963 with the C-terminal portion of TRAIL

The presence of a signal peptide at the N-terminal end of PF963 indicates that this enzyme is secreted via the Sec secretion pathway. The sequence of said signal peptide is SEQ ID NO 36. PCR primers were designed to amplify the DNA sequence corresponding to the promoter and to the signal peptide of protein PF963 (figure 3). Another pair of primers was designed to amplify the sequence of the human cytokine TRAIL. Only the active extracellular sequence Val¹¹⁴-Gly²⁸¹ was amplified. The primer sequences are indicated in the following Table IV.

**Table IV**

| PCR primer | Sequence | Tm (°C) | Total nucleotides | Nucleotides hybridizing with the matrix |
|---|---|---|---|---|
| P963Fw | ATACATATGCCACCGTGAG CTGCACCT | 70 | 27 | 18 |
| | (SEQ ID 38) | | | |
| P963Rv | GCAAGCTTTCGGCCTGTGC AAGTGGTG | 71 | 27 | 19 |
| | (SEQ ID 39) | | | |
| TRAILFw | GCAAGCTTAGTGAGAGAAA GAGGTCCTCAGAGAGTAG | 70 | 37 | 28 |
| | (SEQ ID 40) | | | |
| TRAILRev | ACTGCAGTTAGCCAACTAA AAAGGCCCCGAAAAAACTG G | 70 | 39 | 32 |
| | (SEQ ID 41) | | | |

The construction resulting from the fusion between 1) the promoter and the signal peptide of PF963 and 2) the Val¹¹⁴-Gly²⁸¹ sequence of TRAIL was introduced in cloning vector pPK705 (Kiatpapan *et al.,* 2000). The new pFB4:TRAIL expression plasmid is presented in figure 4.

### III.2.3 Verification of the genetic construction

The sequence of the pFB4:TRAIL plasmid was verified (SEQ ID NO 42). The portion corresponding to the fusion protein ranges from nucleotides 8451 to 9070. This portion is translated in figure 5 (SEQ ID NO 43): the sequence corresponding to the PF963 peptide signal protein is underlined and the Val¹¹⁴-Gly²⁸¹ sequence of TRAIL appears in bold.

The fusion protein has a sequence of 205 amino acid residues corresponding to a mass of 23,190 Da and an isoelectric point of 9.08. The elimination of the signal peptide leads to a sequence of 171 amino acid residues corresponding to a mass of 19,822 Da and an isoelectric point of 8.60.

Expression and secretion of the fusion protein were verified by Western blot using a commercial anti-TRAIL polyclonal antibody (Pab to TRAIL, Alexis Biochemicals). This antibody recognizes the monomeric form (31 kDa) as well as the dimeric form (63 kDa) of TRAIL in the Super*Killer*TRAIL™ preparation (figure 6; lane 4). In the supernatant of the two clones of the transformed *P*. *freudenreichii* strain carrying the plasmid, a protein of 22 kDa, corresponding to the expected size, was detected by this antibody. This protein was absent in the supernatant of the wild strain.

### IV-Development of a second recombinant propionibacterium with the goal of inducing both the intrinsic and extrinsic apoptotic pathways.

The inventors have optimized the plasmid pFB4: TRAIL by changing both the promoter region and the signal peptide to obtain the pCHH04: TRAIL plasmid, allowing secretion into the extracellular medium, of a higher amount of TRAIL into the supernatant.

### IV.1 Materials and methods

The construction of pCHH04:TRAIL is described in Figure 7. The pFB4:TRAIL plasmid, which is a shuttle plasmid, was transformed into *Escherichia coli.* The propionibacterial insert containing the promoter PF963 and the signal peptide PS963 were excised using the appropriate restriction endonucleases. A new propionibacterial DNA fragment (set forth in SEQ ID NO: 69), containing the promoter region and the signal peptide of protein slpA of the *Propionibacterium freudenreichii* CIRM BIA 118 strain, was introduced in order to put the signal peptide in frame with the TRAIL coding sequence. The corresponding sequence was amplified by PCR (PSlpA_fwd_BamH1, which has the sequence set forth in SEQ ID NO: 70 and PS_SlpA-EcoRV-Rev_bis, which has the sequence set forth in SEQ ID NO: 71) from *Propionibacterium freudenreichii* CIRM BIA 1 genomic sequence, which was previously sequenced and annotated (Falentin *et al.,* 2010a). This new plasmid, pCHH04:TRAIL, was then transformed into different strains of *Propionibacterium freudenreichii.* The corresponding supernatants were then analyzed by western blot with respect to TRAIL secretion as above described. Proteomic experiments, including gel electrophoresis and mass spectrometry, was conducted as previously described (Leverrier *et al.,* 2004). TRAIL concentration was further quantified by Enzyme-Linked ImmunoSorbent Assay (ELISA) in supernatant of *Propionibacterium freudenreichii* transformed with pFB4:TRAIL or pCHH04:TRAIL plasmid according to the manufacturer's instructions (R&D System Europe, Lille, France). Human TRAIL standard concentration-response curves were used to quantify TRAIL levels in supernatants. TRAIL concentrations were determined using an automatic plate reader associated with genesis software (LabSystems Spectrophotometer, Cambridge, UK) and data were expressed in ng/ml.

### IV.2 Results

In order to investigate secretion abilities in *Propionibacterium freudenreichii,* supernatants of several strains were analyzed by SDS-PAGE electrophoresis. Figure 8 shows great variability, between strains, in terms of both number and amount of secreted proteins. In particular, *Propionibacterium freudenreichii* CIRM BIA 118 secreted elevated amounts of a 60 kDa protein identified by mass spectrometry as slpA. A translational fusion including slpA signal peptide and TRAIL C-terminal active extracellular part was then obtained (Figure 7, pCHH04:TRAIL). This new construct was compared to pFB4:TRAIL in terms of TRAIL secretion in the CIRM BIA 118 strain. Western blot analysis of the corresponding culture supernatants revealed the presence of a 21 kDa polypeptide identified as TRAIL by immunoblotting. As shown in Figure 9 (left panel), secretion into the extracellular medium of TRAIL was higher using the pCHH04:TRAIL construct, compared to pFB4:TRAIL. Furthermore, different strains of *Propionibacterium freudenreichii* were transformed using pCHH04:TRAIL and secreted TRAIL (Figure 9, right panel). The amount of TRAIL secreted by *Propionibacterium freudenreichii* CIRM BIA 118 (pFB4:TRAIL) was 0.178 ng/ml while it was 22 ng/ml in *Propionibacterium freudenreichii* CIRM BIA 118 (pCHH04:TRAIL) (Table V). This evidences a 100-fold increase in secretion efficiency. Moreover, similar secretion efficiency was demonstrated in different strains of *Propionibacterium freudenreichii* (including CIRM BIA 512, 125, 118, 129 and 122 strains, Figure 9 right panel and Table V).

**Table V - ELISA quantification of human TRAIL in Propionibacterium _freudenreichii supernatants (corresponding to supernatants analysed by western blot and shown in Figure 9. left panel)**

| ELISA | Human soluble TRAIL (ng/ml) |
|---|---|
| CIRM BIA 118 WT | 0 |
| CIRM BIA 118 pFB4:TRAIL | 0.178 |
| CIRM BIA 118 pFB4 | 0 |
| CIRM BIA 118 pCHH04:TRAIL | 22 |
| CIRM BIA 122 pCHH04:TRAIL | Non Determined |
| CIRM BIA 125 pCHH04:TRAIL | 24 |
| CIRM BIA 129 pCHH04:TRAIL | 22 |
| CIRM 512 pCHH04:TRAIL | Non Determined |
| CIRM 118 pCHH04:TRAIL | 23 |

### IV.3 Discussion

The expression and secretion construction pCHH04: TRAIL allows higher amounts of TRAIL secretion into the extracellular medium than the pFB4: TRAIL construction. The slpA promoter, isolated from strain CIRM BIA 118, is most probably stronger than PF963 promoter. The slpA signal peptide allows efficient secretion of the TRAIL human cytokine in different *Propionibacterium strains.* Other slp proteins were then identified from the genomic sequence of several *Propionibacterium freudenreichii* strains. The corresponding signal peptides are presented in Table III and may also be used to allow heterologous protein expression in Bacteria, in particular belonging to the order *Actinomycetales,* in particular to the genus *Propionibacterium,* and more particularly to the species *Propionibacterium freudenreichii.*

### REFERENCES

- Ashkenazi A et al. 1999. J. Clin. Invest. 104:155-162.
- Bougle et al. 1999. Scand. J. Gastroenterol. 34:144-148.
- Deutsch et al. 2012. Applied and Environmental Microbiology 78: 1765-1775
- Emanuelsson et al. 2007. Nature Protocols 2:953-971.
- Falentin et al. 2010a. Plos One 5:e11748.
- Falentin et al. 2010b. Int. J Food Microbiol. 144 :10-19.
- Ganai et al. 2009. British Journal of Cancer. 101:1683-1691.
- Hervé et al. 2007. Int J Food Microbiol. 113:303-314.
- Hu et al. 2009. Cancer Gene Therapy. 16:655-663.
- Jan et al. 2002. Cell Death Differ. 9:179-188.
- Kiatpapan et al. 2000. Appl. Environ. Microbiol. 66:4688-4695.
- Lan et al. 2007a. Apoptosis 12:573-591.
- Lan et al. 2007b. Br J Nutr. 97:714-724.
- Lan et al. 2008. Br J Nutr. 100:1251-1259.
- Lacour et al. 2001. Cancer Res. 61:1645-1651.
- Lacour et al. 2003. Oncogene 22:1807-1816.
- Leverrier et al. 2004. Arch. Microbiol. 181 :215-230.
- Lortal et al. 1993. Applied and Environmental Microbiology 59(8): 2369.
- Meurette et al. 2005. Clin. Cancer Res. 11:3075-3083.
- Meurette et al. 2006. Ann N Y Acad Sci. 1090:209-216.
- Meurette et al. 2007. Cancer Res. 67:218-226.
- Patch J.A. and Barron A.E., 2002. Curr. Opin. Chem. Biol., 6(6):872-877. Review.
- Sträter J. et al. 2002. Gastroenterology 122:659-666.
- Sambrook and Russell (2001) Molecular Cloning: A Laboratory Manual (3rd Ed.) Cold Spring Harbor Laboratory Press, Cold Spring Harbor, N.Y.
- Tarze et al. 2007. Oncogene 26:2606-2620.
- Zarate et al. 2000. J. Food Prot. 63:1214-1221.
- Zhang et al. 2010. Cancer Gene Therapy 17:334-343

## Claims

1. A vector comprising:
- at least one promoter operatively linked to
- at least one nucleic acid sequence encoding a signal peptide translationally fused to
- at least one nucleic acid sequence encoding a substance of interest;
■ wherein said nucleic acid sequence encoding a signal peptide is selected from the group consisting of:
- the sequence of the signal peptide of a gene encoding a surface layer protein of a *Propionibacterium freudenreichii;* and
- the sequences having at least 80% of identity with said sequence over the entire length of said sequence;
and/or
■ wherein the sequence of said promoter is selected from the group consisting of:
- the sequence of the promoter of a gene encoding a surface layer protein of a *Propionibacterium freudenreichii;* and
- the sequences having at least 80% of identity with said sequence over the entire length of said sequence.

2. The vector according to claim 1, wherein said nucleic acid sequence encoding a signal peptide is selected from the group consisting of:
- the sequence of the signal peptide of a gene encoding a surface layer protein of a *Propionibacterium freudenreichii;* and
- the sequences having at least 80% of identity with said sequence over the entire length of said sequence;
and wherein the sequence of said promoter is selected from the group consisting of:
- the sequence of the promoter of a gene encoding a surface layer protein of a *Propionibacterium freudenreichii;* and
- the sequences having at least 80% of identity with said sequence over the entire length of said sequence.

3. The vector according to claim 1 or 2, wherein said surface layer protein is selected from the group consisting of the surface layer protein A, the surface layer protein B, the surface layer protein C, the surface layer protein D, the surface layer protein E and the surface layer protein F.

4. The vector according to claim 3, wherein said nucleic acid sequence encoding a signal peptide is selected from the group consisting of:
- the nucleic acid sequences encoding the signal peptides of sequences SEQ ID NO: 59 to 68; and
- the sequences having at least 80% of identity with one of said sequences over the entire length of said sequence.

5. The vector according to claim 4, comprising the nucleic acid sequence SEQ ID NO: 69 or a sequence having at least 80% of identity with SEQ ID NO: 69 over the entire length of said sequence, translationally fused to said nucleic acid sequence encoding a substance of interest.

6. The vector according to any one of claims 1 to 5, wherein said substance of interest is a peptide or a protein chosen in the group consisting of antibodies, receptor ligands, hormones, cytokines, growth factors, cell adhesion molecules, blood clotting factors, enzymes, fragments thereof and combinations thereof, more particularly in the group consisting of cytokines, antibodies and hormones, and even more particularly from the group consisting of proapoptotic TRAIL protein and the C-terminal extracellular domain of the TRAIL protein.

7. The vector according to claim 6, wherein said substance of interest is the C-terminal extracellular domain of the TRAIL protein consisting of the sequence from amino acids 114 to 281 of the sequence set forth in SEQ ID NO: 58.

8. A bacterium comprising a vector according to any one of claims 1 to 7.

9. The bacterium according to claim 8, which belongs to the order *Actinomycetales,* in particular to the genus *Propionibacterium,* and more particularly to the species *Propionibacterium freudenreichii.*

10. The propionibacterium deposited on November 13, 2012 under number CNCM I-4692 with the *Collection Nationale de Cultures de Microorganismes* (CNCM, Institut Pasteur, 25 rue du Docteur Roux, 75724 Paris Cedex 15, France).

11. A composition comprising at least one vector according to any one of claims 1 to 7 and/or at least one bacterium according to any one of claims 8 to 10.

12. A vector according to any one of claims 1 to 7 or a bacterium according to any one of claims 8 to 10 for use as a medicament.

13. A vector according to any one of claims 1 to 5 or a bacterium according to any one of claims 8 to 10 for use in the prevention and/or treatment of cancer; wherein said substance of interest is the proapoptotic TRAIL protein or the C-terminal extracellular domain of the TRAIL protein.

14. The bacterium for use according to claim 13, said bacterium belonging to the genus *Propionibacterium,* particularly to the species *Propionibacterium freudenreichii.*

15. A product comprising:
- at least one vector according to any one of claims 1 to 7 and/or at least one bacterium according to any one of claims 8 to 10; and
- at least another active agent, in particular chosen in the group consisting of an anti-tumoral agent, an anti-inflammatory agent and an immunomodulatory agent;
as a combination product for simultaneous, separate or sequential use for the prevention and/or treatment of at least one disease, in particular selected from the group consisting of allergies, hypertension, obesity, cancers and inflammatory colon diseases.

16. A method for producing and secreting into the extracellular medium at least one substance of interest, by at least one bacterium as defined in any one of claims 8 to 10, said method comprising the steps of:
- culturing said bacterium under suitable conditions; and
- recovering the culture medium containing said substance of interest.

17. An isolated nucleic acid molecule of sequence selected from the group consisting of:
- the nucleic acid sequences encoding the peptides of sequences SEQ ID NO: 59 to 68;
- the nucleic acid sequence SEQ ID NO: 69; and
- the sequences having at least 80% of identity with one of said sequences over the entire length of said sequence.


SEQUENCE LISTING

<110>  UNIVERSITE DE RENNES I
       INSTITUT NATIONAL DE LA RECHERCHE AGRONOMIQUE
       INSTITUT NATIONAL DE LA SANTE ET DE LA RECHERCHE MEDICALE
       (INSERM)

<120>  VECTORS FOR PRODUCING AND SECRETING SUBSTANCE OF INTEREST BY
       BACTERIA AND APPLICATIONS THEREOF

<130>  70609D27981

<160>  71

<170>  PatentIn version 3.5

<210>  1
<211>  99
<212>  DNA
<213>  Propionibacterium freudenreichii

<400>  1
atgtcaaaga cactctctcg gatcgcatcc gtcgcttcgg ttgccgcgct cgccggcagc      60

atcaccgtca tcgccgggca gaacgcgtcc gccgacagc                             99


<210>  2
<211>  90
<212>  DNA
<213>  Propionibacterium freudenreichii

<400>  2
gtgaagaacg gtctcaagac cctgctcatt ggtggagtcg gcatcgcgac ccttgcggtc      60

ggcggcgtcg gaactgccat cgcagacaat                                       90


<210>  3
<211>  105
<212>  DNA
<213>  Propionibacterium freudenreichii

<400>  3
atgcgatcca ccacgacgaa ggcgtttgcc ggtgtcgctg tgctggcgct ggcgcttgct      60

ggctgcggct cgaattcggg ctcgtccacc aagtcggccg acagc                     105


<210>  4
<211>  93
<212>  DNA
<213>  Propionibacterium freudenreichii

<400>  4
atgctcactc gcaagagagt ggttgcagcg ggagctgccg ccaccctgtc cctcacggcg      60

tttgccgggt tgcagcccgc cagcgccgcc acc                                   93


<210>  5
<211>  108
<212>  DNA

<213>  Propionibacterium freudenreichii

<400>  5
atgggattca gggttggccg tcgtcccctc atcggggcag ttctcgccgg gtcgatggca      60

acactcgtgg gctgttccac ctcgggtagc ggcagtggag cctccagc                  108


<210>  6
<211>  111
<212>  DNA
<213>  Propionibacterium freudenreichii

<400>  6
atgcaggccc tccaaggaag gcgccggtca cgacgggtga tggcggccgc ggtagcagcc      60

ctcaccgcca tgaccgtgct gccctcccag ctcaacgccg ttgctgcacc c              111


<210>  7
<211>  90
<212>  DNA
<213>  Propionibacterium freudenreichii

<400>  7
atgtccactg gccgcatgaa gttcatcaag ctggcagttc ctgtcatcgt tgcctgctgc      60

ttgacgccaa tggctgcctt agctgatgtg                                      90


<210>  8
<211>  114
<212>  DNA
<213>  Propionibacterium freudenreichii

<400>  8
atggcgatga gggcacgtca cggcgtcgtc cggcttggtc tggtctgtct caccgcattg      60

gcggtcttcg gcacggcaaa tgtgtcgggt caggttgcgg tgatggctga gggc          114


<210>  9
<211>  108
<212>  DNA
<213>  Propionibacterium freudenreichii

<400>  9
ttgaaccagg ccctgtcgac gatgcgcctg aagatcggcg actccaccaa gcgcatccgg      60

atcttcttcg tcgtgatggc cgtggcgatc accctgctcg cgggacgg                  108


<210>  10
<211>  75
<212>  DNA
<213>  Propionibacterium freudenreichii

<400>  10
atgcgacgtc gcaccacgat tgcagccctc gctgctgtct tgagtttcag tcccctggcc      60

gcccaggccg caccc                                                      75

<210>  11
<211>  132
<212>  DNA
<213>  Propionibacterium freudenreichii

<400>  11
atgcctagtc atgcggtgcg ggagacgcgg gcgaacaagt tgcgccggtt cctgcggccc      60

accgttgccc agggcgtgct cggtatcgcg ttctgcctcg tggccgccgt cggcgtggtg     120

cagatccgct cc                                                         132


<210>  12
<211>  111
<212>  DNA
<213>  Propionibacterium freudenreichii

<400>  12
atgtcacgga ttcaactccc ccggctgagc cggattgcga tcgcagcagc agcttccgct      60

gccctgatcg gcaccagctt catcgccccg gccacggcct ttgccgcgcc g              111


<210>  13
<211>  75
<212>  DNA
<213>  Propionibacterium freudenreichii

<400>  13
ctagccgacc ttctcggcct tgctggccag gtcgtcgggg atcgtgaccc ccatcaggga      60

ggcggccttc tcatt                                                       75


<210>  14
<211>  120
<212>  DNA
<213>  Propionibacterium freudenreichii

<400>  14
ttagttgttg gggacgagga gggagtggag ttcgatgacg tcgagggtgg gtgcggtggc      60

ggggcgggtg atggtttcgg tgccggtgtg tccttgggcg gtccaggtga tggtccaggt     120


<210>  15
<211>  60
<212>  DNA
<213>  Propionibacterium freudenreichii

<400>  15
tcagccagtt ggtgccggcc ttggcgtcgg cggcgtggga tacacgcgga actgggcgcc      60


<210>  16
<211>  114
<212>  DNA
<213>  Propionibacterium freudenreichii

<400>  16
atgttcattt cgcgcttccg tcgtgcggct gcggtcggcc tggccgccgt caccgcattg      60

tccgccactg cctgtagcgg ttcctcgtcg tcgtccagct catccgcgag ctcg 114

<210> 17
<211> 108
<212> DNA
<213> Propionibacterium freudenreichii

<400> 17
atgaagtccg cgacgcgacg cccgctgacg cgctggattg tcgccttcgg ggtggtgttg 60

gtgctggtca tcgccgggtc ggtggggctg catgcctccg gtgccctg 108

<210> 18
<211> 108
<212> DNA
<213> Propionibacterium freudenreichii

<400> 18
gtgaatccct tcgtcaagac ggcgcgcgtg gctatcacct cgacgctggt ggcaggctcg 60

ctcgccactg ccagcctcgt gtttgcacca cttgcacagg ccgattac 108

<210> 19
<211> 33
<212> PRT
<213> Propionibacterium freudenreichii

<400> 19

Met Ser Lys Thr Leu Ser Arg Ile Ala Ser Val Ala Ser Val Ala Ala
1               5                   10                  15

Leu Ala Gly Ser Ile Thr Val Ile Ala Gly Gln Asn Ala Ser Ala Asp
            20                  25                  30

Ser

<210> 20
<211> 30
<212> PRT
<213> Propionibacterium freudenreichii

<400> 20

Met Lys Asn Gly Leu Lys Thr Leu Leu Ile Gly Gly Val Gly Ile Ala
1               5                   10                  15

Thr Leu Ala Val Gly Gly Val Gly Thr Ala Ile Ala Asp Asn
            20                  25                  30

<210> 21
<211> 35
<212> PRT
<213> Propionibacterium freudenreichii

<400>  21

Met Arg Ser Thr Thr Thr Lys Ala Phe Ala Gly Val Ala Val Leu Ala
1               5                   10                  15

Leu Ala Leu Ala Gly Cys Gly Ser Asn Ser Gly Ser Ser Thr Lys Ser
            20                  25                  30

Ala Asp Ser
        35

<210>  22
<211>  31
<212>  PRT
<213>  Propionibacterium freudenreichii

<400>  22

Met Leu Thr Arg Lys Arg Val Val Ala Ala Gly Ala Ala Ala Thr Leu
1               5                   10                  15

Ser Leu Thr Ala Phe Ala Gly Leu Gln Pro Ala Ser Ala Ala Thr
            20                  25                  30

<210>  23
<211>  36
<212>  PRT
<213>  Propionibacterium freudenreichii

<400>  23

Met Gly Phe Arg Val Gly Arg Arg Pro Leu Ile Gly Ala Val Leu Ala
1               5                   10                  15

Gly Ser Met Ala Thr Leu Val Gly Cys Ser Thr Ser Gly Ser Gly Ser
            20                  25                  30

Gly Ala Ser Ser
        35

<210>  24
<211>  37
<212>  PRT
<213>  Propionibacterium freudenreichii

<400>  24

Met Gln Ala Leu Gln Gly Arg Arg Arg Ser Arg Arg Val Met Ala Ala
1               5                   10                  15

Ala Val Ala Ala Leu Thr Ala Met Thr Val Leu Pro Ser Gln Leu Asn
            20                  25                  30

Ala Val Ala Ala Pro
        35

<210>  25
<211>  30
<212>  PRT
<213>  Propionibacterium freudenreichii

<400>  25

Met Ser Thr Gly Arg Met Lys Phe Ile Lys Leu Ala Val Pro Val Ile
1               5                   10                  15

Val Ala Cys Cys Leu Thr Pro Met Ala Ala Leu Ala Asp Val
            20                  25                  30

<210>  26
<211>  38
<212>  PRT
<213>  Propionibacterium freudenreichii

<400>  26

Met Ala Met Arg Ala Arg His Gly Val Val Arg Leu Gly Leu Val Cys
1               5                   10                  15

Leu Thr Ala Leu Ala Val Phe Gly Thr Ala Asn Val Ser Gly Gln Val
            20                  25                  30

Ala Val Met Ala Glu Gly
        35

<210>  27
<211>  36
<212>  PRT
<213>  Propionibacterium freudenreichii

<400>  27

Met Asn Gln Ala Leu Ser Thr Met Arg Leu Lys Ile Gly Asp Ser Thr
1               5                   10                  15

Lys Arg Ile Arg Ile Phe Phe Val Val Met Ala Val Ala Ile Thr Leu
            20                  25                  30

Leu Ala Gly Arg
        35

<210>  28
<211>  25
<212>  PRT
<213>  Propionibacterium freudenreichii

<400>  28

Met Arg Arg Arg Thr Thr Ile Ala Ala Leu Ala Ala Val Leu Ser Phe
1               5                   10                  15

Ser Pro Leu Ala Ala Gln Ala Ala Pro
            20                  25

<210>  29
<211>  44
<212>  PRT
<213>  Propionibacterium freudenreichii

<400>  29

Met Pro Ser His Ala Val Arg Glu Thr Arg Ala Asn Lys Leu Arg Arg
1               5                   10                  15

Phe Leu Arg Pro Thr Val Ala Gln Gly Val Leu Gly Ile Ala Phe Cys
            20                  25                  30

Leu Val Ala Ala Val Gly Val Val Gln Ile Arg Ser
        35                  40

<210>  30
<211>  37
<212>  PRT
<213>  Propionibacterium freudenreichii

<400>  30

Met Ser Arg Ile Gln Leu Pro Arg Leu Ser Arg Ile Ala Ile Ala Ala
1               5                   10                  15

Ala Ala Ser Ala Ala Leu Ile Gly Thr Ser Phe Ile Ala Pro Ala Thr
            20                  25                  30

Ala Phe Ala Ala Pro
        35

<210>  31
<211>  25
<212>  PRT
<213>  Propionibacterium freudenreichii

<400>  31

Met Lys Arg Arg Thr Leu Leu Gly Thr Leu Gly Ile Met Gly Leu Ser
1               5                   10                  15

Val Pro Leu Ala Ala Cys Ser Ser Lys
            20                  25

<210>  32
<211>  40
<212>  PRT
<213>  Propionibacterium freudenreichii

<400>  32

Met Val Thr Gly Gly Asn Asp Met Pro Ser Lys Arg Ile Thr Thr Trp
1               5                   10                  15

Pro Gly Ile Ser Ala Leu Ser Ala Leu Ile Ala Gly Met Leu Leu Ala
            20                  25                  30

Pro Leu Pro Val Ala Ala Asp Gly
        35                  40

<210>  33
<211>  20
<212>  PRT
<213>  Propionibacterium freudenreichii

<400>  33

Met Ala Met Val Met Ala Ser Leu Ala Met Phe Gly Ala Ser Arg Ala
1               5                   10                  15

Ser Ala Ala Asp
            20

<210>  34
<211>  38
<212>  PRT
<213>  Propionibacterium freudenreichii

<400>  34

Met Phe Ile Ser Arg Phe Arg Arg Ala Ala Ala Val Gly Leu Ala Ala
1               5                   10                  15

Val Thr Ala Leu Ser Ala Thr Ala Cys Ser Gly Ser Ser Ser Ser Ser
            20                  25                  30

Ser Ser Ser Ala Ser Ser
        35

<210>  35
<211>  36
<212>  PRT
<213>  Propionibacterium freudenreichii

<400>  35

Met Lys Ser Ala Thr Arg Arg Pro Leu Thr Arg Trp Ile Val Ala Phe
1               5                   10                  15

Gly Val Val Leu Val Leu Val Ile Ala Gly Ser Val Gly Leu His Ala
20                  25                  30

Ser Gly Ala Leu
        35

<210>  36
<211>  36
<212>  PRT
<213>  Propionibacterium freudenreichii

<400>  36

Met Asn Pro Phe Val Lys Thr Ala Arg Val Ala Ile Thr Ser Thr Leu
1               5                   10                  15

Val Ala Gly Ser Leu Ala Thr Ala Ser Leu Val Phe Ala Pro Leu Ala
            20                  25                  30

Gln Ala Asp Tyr
        35

<210>  37
<211>  572
<212>  PRT
<213>  Propionibacterium freudenreichii

<400>  37

Met Asn Pro Phe Val Lys Thr Ala Arg Val Ala Ile Thr Ser Thr Leu
1               5                   10                  15

Val Ala Gly Ser Leu Ala Thr Ala Ser Leu Val Phe Ala Pro Leu Ala
            20                  25                  30

Gln Ala Asp Tyr Ser Pro Leu Ala Ala Thr Ala Thr Val Asn Val Arg
        35                  40                  45

Gln Gly Pro Asp Thr Ser Ser Ser Val Leu Ala Thr Leu Ser Ser Gly
    50                  55                  60

Asp Thr Val Thr Gln Arg Gly Ala Glu Gln Asp Gly Trp Leu Pro Ile
65                  70                  75                  80

Thr Tyr Asn Gly Ala Asn Ala Trp Ile Gln Ala Gln Tyr Val Ala Ser
                85                  90                  95

Thr Thr Ala Ala Thr Gln Lys Asp Gln Ile Ser Thr Ala Glu Leu Thr
            100                 105                 110

Ala Asp Ala Tyr Val Arg Thr Ala Ala Asn Ala Asn Ala Trp Val Leu
        115                 120                 125

Gly Thr Ala His Thr Gly Asp Lys Val Gly Ile Thr Gly Gln Ala Ser
    130                 135                 140

Gly Asp Tyr Thr Pro Val Asn Phe Tyr Gly Arg Ala Gly Trp Ile Ala
145                 150                 155                 160

Thr Lys Leu Leu Ser Ala Ala Asp Ala Ser Val Thr Ser Ile Lys Ile
                165                 170                 175

Thr Thr Ala Ile Ser Ser Asp Tyr Leu Trp Val Arg Gly Gly Glu Ser
            180                 185                 190

Thr Ala Ala Gln Ser Ile Gly Met Leu Tyr Pro Gly Asp Arg Val Asp
        195                 200                 205

Val Thr Gly Asp Pro Val Gly Gly Trp Val Pro Ile Asn Phe Asn Gly
    210                 215                 220

Lys Thr Ala Phe Val Ala Ala Asn Tyr Ser Arg Tyr Leu Thr Asp Pro
225                 230                 235                 240

Thr Val Val Thr Leu Ser Thr Lys Thr Asp Val Thr Asn Lys Asp Thr
                245                 250                 255

Ala Thr Ser Thr Gly Thr Asp Ser Ser Thr Ala Gly Gly Ser Thr Ala
            260                 265                 270

Thr Thr Pro Thr Thr Thr Ala Pro Thr Thr Thr Ala Pro Ala Thr Lys
        275                 280                 285

Pro Thr Thr Thr Pro Pro Ala Thr Thr Gln Ala Ala Ala Ser Thr Lys
    290                 295                 300

Tyr Thr Thr Ala Asp Val Asn Val Arg Val Gly Pro Gly Ile Asp Gln
305                 310                 315                 320

Gln Pro Val Thr Val Leu Lys Glu Asn Ser Gln Val Ala Ala Thr Gly
                325                 330                 335

Lys Thr Ser Gly Asp Trp Thr Glu Val Ser Tyr Asp Gly Ala Ser Arg
            340                 345                 350

Trp Ile Ser Ser Gln Tyr Leu Ser Asp Thr Lys Gln Ala Glu Ala Pro
        355                 360                 365

Ala Pro Ala Pro Ala Pro Asp Pro Thr Pro Ala Gly Pro Thr Gly Ser
    370                 375                 380

Arg Trp Thr Thr Ala Ala Leu Asn Ala Tyr Gly Ser Ser Thr Gln Pro
385                 390                 395                 400

Lys Pro Ala Thr Thr Val Val Pro Glu Gly Thr Gln Val Glu Leu Thr
                405                 410                 415

Gly Lys Gln Ala Asp Gly Arg Ser Glu Tyr Thr Trp Asn Gly Thr Thr
            420                 425                 430

Tyr Trp Ser Ala Thr Glu Tyr Leu Gly Thr Asn Ala Pro Ala Thr Asn
        435                 440                 445

Thr Ser Ala Asn Thr Ala Lys Pro Gly Ala Asn Ala Val Glu Thr Ala
    450                 455                 460

Ile Asn Phe Ala Met Ser Lys Leu Gly Gly Pro Tyr Val Trp Gly Gly
465                 470                 475                 480

Thr Gly Pro Val Gly Tyr Asp Cys Ser Gly Leu Met Gln Ala Ala Tyr
                485                 490                 495

Ala Ala Ala Gly Val Thr Leu Pro Arg Val Thr Trp Asp Gln Val Asn
            500                 505                 510

Ala Gly Lys Gln Val Ser Val Gly Asp Leu Gln Pro Gly Asp Leu Val
        515                 520                 525

Phe Phe Tyr Asp Asn Gly His Val Gly Met Tyr Ile Gly Asn Gly Asn
    530                 535                 540

Ile Val Asn Ala Leu Asn Glu Asp Ala Gly Ile Val Val Thr Pro Ile
545                 550                 555                 560

Ser Tyr Met Pro Ile Ser Ala Ala Val Arg Ile Ala
                565                 570

<210>  38
<211>  27
<212>  DNA
<213>  artificial

<220>
<223>  Primer P963Fw

<400>  38
atacatatgc caccgtgagc tgcacct                                          27

<210> 39
<211> 27
<212> DNA
<213> artificial

<220>
<223> Primer P963Rv

<400> 39
gcaagctttc ggcctgtgca agtggtg 27


<210> 40
<211> 37
<212> DNA
<213> artificial

<220>
<223> Primer TRAILFw

<400> 40
gcaagcttag tgagagaaag aggtcctcag agagtag 37


<210> 41
<211> 39
<212> DNA
<213> artificial

<220>
<223> Primer TRAILRv

<400> 41
actgcagtta gccaactaaa aaggccccga aaaaactgg 39


<210> 42
<211> 9073
<212> DNA
<213> artificial

<220>
<223> Sequence of the plasmid pFB4:TRAIL

<400> 42
ggtcgactct agagggatcc ggcggaactt cacgtcctgg cggtggagtt ggcgggcgcg 60

ttccagccgt tcctccagca cggtgatccg ggcctccaga cgctcacgct caccctgctc 120

caggtgccgg gtcaccgtca ccgtccgcac cggccgggcc tcggcctggg cggcccggcg 180

ttcctcactg gcccgcttcc ggcaatcgtc ggaacaccac acccggggcc gaccccgccc 240

accgtgggcc tccaccggcg ccccgcagtg gggacacgcc cgcagcgccg acgcatcctc 300

atccaaggcc atcaccgggt cggaatccat acccgaaacc atatcgtccg gacgatgaac 360

tgcgccagac agctaagaat gcacgaggtg tgtctccgat tctcaggaaa cgctcagcat 420

tttccgagac gttcggcgca cgcacacacc cccacaagaa ccgacccgcc cagcatccgc 480

cgacacgtcg atccgcaccc gcgatgggct ggccgaggcc gactacgacc gctagtcagc 540

acctgcgctg atctaccgtc gccctgaccg actctcccgt cgggattgtc gccggccgct 600

gccagcatgg acctgcggcc ccgccccctc gccctgcaac tcgagggagg cggggccgtc 660

caccccccac accaccccga caccgtgatg cgcccatgtc gcctaacggg ttgcccgacc 720

tccccgacat caagaaaacc tgacaccgtc gccgcaagcg ctacactgac tactagtagt 780

caggaggtgc gtgatgacca tcgccacatc ggtgaaactc tccgaagaga ccggccgcaa 840

actcgatgaa ctagcccggg ccaccgggcg atccaagtcc tactacctgc gcgaggccat 900

cgaggaccac atcgaccaga tggtccacga ctacgccatc gcccgactcg ccgacgacgt 960

gcgagccggc cgggccgcca cctacagcgc cgacgaagtg gaccagatcc ttggcctgga 1020

cgattgagta caccgacccc gccgtcaaag cactgcgcaa actcgaccga gcccaggccc 1080

gccgcatcac cgcctacata cgtgagctca ccggcctgga cgatccccac caacgcggga 1140

aaggaaaata aaaaagggga cctctagggt ccccaattaa ttagtaatat aatctattaa 1200

aggtcattca aaaggtcatc caccggatca attcccctgc tcgcgcaggc tgggtgccaa 1260

gctctcgggt aacatcaagg cccgatcctt ggagcccttg ccctcccgca cgatgatcgt 1320

gccgtgatcg aaatccagat ccttgacccg cagttgcaaa ccctcactga tccgtaatgt 1380

gagttagctc actcattagg caccccaggc tttacacttt atgcttccgg ctcgtatgtt 1440

gtgtggaatt gtgagcggat aacaatttca cacaggaaac agctatgacc atgattacgc 1500

caagctgtgg cgtacaccgt cgcctcggtc ggcccgtaga gattggcgat cccgaccgca 1560

gcaccaccga gaacgtcccc gacgtggccg accagcccgt catcgtcaac gcctgaccgc 1620

ggtgcggaca ggccgtgtcg cgaccggccg tgcggaatta agccggcccg taccctgtga 1680

atagaggtcc gctgtgacac aagaatccct gttacttctc gaccgtattg attcggatga 1740

ttcctacgcg agcctgcgga acgaccagga attctgggag ccgctggccc gccgagccct 1800

ggaggagctc gggctgccgg tgccgccggt gctgcgggtg cccggcgaga gcaccaaccc 1860

cgtactggtc ggcgagcccg acccggtgat caagctgttc ggcgagcact ggtgcggtcc 1920

ggagagcctc gcgtcggagt cggaggcgta cgcggtcctg gcggacgccc cggtgccggt 1980

gccccgcctc ctcggccgcg gcgagctgcg gcccggcacc ggagcctggc cgtggcccta 2040

cctggtgatg agccggatga ccggcaccac ctggcggtcc gcgatggacg gcacgaccga 2100

ccggaacgcg ctgctcgccc tggcccgcga actcggccgg gtgctcggcc ggctgcacag 2160

ggtgccgctg accgggaaca ccgtgctcac cccccattcc gaggtcttcc cggaactgct 2220

gcgggaacgc cgcgcggcga ccgtcgagga ccaccgcggg tggggctacc tctcgccccg 2280

gctgctggac cgcctggagg actggctgcc ggacgtggac acgctgctgg ccggccgcga 2340

accccggttc gtccacggcg acctgcacgg gaccaacatc ttcgtggacc tggccgcgac 2400

cgaggtcacc gggatcgtcg acttcaccga cgtctatgcg ggagactccc gctacagcct 2460

ggtgcaactg catctcaacg ccttccgggg cgaccgcgag atcctggccg cgctgctcga 2520

cggggcgcag tggaagcgga ccgaggactt cgcccgcgaa ctgctcgcct tcaccttcct 2580

gcacgacttc gaggtgttcg aggagacccc gctggatctc tccggcttca ccgatccgga 2640

ggaactggcg cagttcctct gggggccgcc ggacaccgcc cccggcgcct gacgccccgg 2700

gcctaccgct gacacgcaac cccgcaccct cggccaagac gtcacacacc acccgcccca 2760

ccgagcactg aggatgtcaa ctcgcccgag ccggcctgcc ggccgtctta cgggttgtct 2820

tggcgggcgg ggtgtctttg ccctggccca gcagccccac gatctcccgc agcgtgtcgg 2880

cggtggcggc gtcccgggcc gcctgacgct ccgcctccgc cctggcctgc tcggctgcct 2940

gcgcccgatc ctccgcggcg gcggcctgct ccctcgcctc ggccagctcg ccggtcaggg 3000

cctcgacccg ggcctgcacc tgccccaggc gcgcctccgc ctcctgctgc acctgctcgg 3060

cccgggcctc cgcctggtcc cgggccgcct cggcctcggc ccggtgctga tccgccaggg 3120

ccgcctcggc caccgcttcg gcctgcccat ccaccgcctg ctcggcccga gccccgaact 3180

cctcgcgggc cgcatcactc gcctgacgcc acgccgccgc ccacaccaga cccaacggct 3240

ccgacagatc cggcggggcc ggcgtctgga ccgacgccga gacgtcgcgc aggaaccccg 3300

ccgcagcgtc ggtggagcac cccgcctccg ccttcaacga ccgcaccgtc acccgccgac 3360

ccgcaccgct caaccgcgca taggccgccg ccaaccttga cccattcgac tccatgaccc 3420

accctcccat tctgtaccct gtacctgttc ctaggtacgt tcctaatgta cctcaccgga 3480

tgcagaaccc gcaacccccc tcacactccc cctgcacggg gcccgccccc tgcacccccg 3540

ctgccgcgcc cgctcctgcg tcgcggcctt gcccctgccc aacgccgggc cggcgggcag 3600

cccaccagag gctctgtgag acgtcggcgc ccccgtccac ctaccctaaa gaccaaccgg 3660

ccgtggaaac gtctgtgagg agccttgtag gagttcccag gacaagccag caaggccggg 3720

cctgacggcc cggaaaggaa gtcgctgcgc tcctacgaag aagcccctct ggggaccccc 3780

agaccccgga actatctgat ttggtttagc ggcgtacttc cgtcataccg gaatttatgg 3840

catgctgtgg tcatggcgac gacgacggtc gatgagcagt gggagcaggt gtggctgccc 3900

cgctggcccc tggcctccga cgacctggca gcgggcatct accggatggc ccgcccctcg 3960

gcgctggggg tccgatacat cgaggtcaac ccccaagcca tcagcaacct cctcgtggtc 4020

gactgcgacc accccgacgc tgccatgcgc gccgtctggg accgccacga ctggctgccc 4080

aacgccatcg tcgagaaccc cgacaacggc cacgcccacg ccgtgtgggc cctggaagca 4140

gccatcccgc gcaccgagta cgcccaccgc aagcccatcg cctacgccgc cgccgtcacc 4200

gagggcctgc gccgatccgt cgacggagac gcctcctacg ccggcctgat caccaagaac 4260

cccgaacacc ccgcctggaa caccacctgg tgcaccgacc acctctaccg gctggccgag 4320

ctcgacaccc acctggatgc cgccggcctc atgcccgccc cctcctggcg acgcacccgc 4380

cggcgcaacc ccgtcggcct gggccgcaac tgcgccatct tcgagaccgc ccgcacctgg 4440

gcctaccgcg acgcccgccg catccgacaa cgccacgaat acccgaccgc cgaggactcg 4500

gccgacctgc acgccgtcat cgcctccacc gtcgaggcgc tcaacgccgg ctacagcgaa 4560

cccctgccgg cccgcgaggc cgccggcatc gccgccagca tccaccgatg gatcacccac 4620

cgtttctacg gctggatcga ctcccacacc gtcaacgagg ccactttctc caccatccag 4680

agctacagag gacacaaggg agccggcaag gctcgtcctc gtgcccgccg tgctgcttct 4740

atcaccgatt gggaggcatg atggctgacg tccagcaccg cgtgaagcgt cggggcacgg 4800

cccgcgaggc cgcagaacgt gtaggggcct ccatccgaac cgcccagcgg tggacctcca 4860

tcccccgtga ggaatggatc actcagaagg ccgtcgagcg tgaggagatc cgggcctaca 4920

agtacgacga ggggcacacg tggggcgaga cctcgcgcca cttcgggatc gcgaagacca 4980

ccgcccagga gcgggcccgg cgggctcgaa gggagcgggc ggccgaagcg gagaaggctg 5040

ccgaggaggc cgaggccgcg ctgcgtccga cactcttcga gggccaggag caaggttctg 5100

catgagcaac cccgagtcct cgggtagacc gtctggcccg acgttaagca tggctgaagc 5160

ggcccgtgcc tgtggggttt cagtgtccac ggtgaggcgt caccgtgatg ccctggtggc 5220

ccacggtgct acccgtcatg acgcgtcatg ggtgataccc ctatcagcgt tgatttcatg 5280

cggtttgatg ccccgggtga caccccctga tgccccgtca cccaataacg tggcgcctgc 5340

catgacgtcc cacggtgacg cccccctgac gggggaagtc caagagctgc gcgagcgact 5400

ggccaacgct gagcatcgag ccgagctagc agtagaggtt ggggacgacg tctcggcgac 5460

tccggagaac accaagtcag ggtctcatga gtgtgcgata gcttgagctg tctaccaatc 5520

tggatatagc tatatcggtc gtttgtgtct gattcgccag tgagccaacg gcgggggcga 5580

cacgcggtgg cgaaaccccc tggcagaatt cgtaatcatg gtcatagctg tttcctgtgt 5640

gaaattgtta tccgctcaca attccacaca acatacgagc cggaagcata aagtgtaaag 5700

cctggggtgc ctaatgagtg agctaactca cattaattgc gttgcgctca ctgcccgctt 5760

tccagtcggg aaacctgtcg tgccagctgc attaatgaat cggccaacgc gcggggagag 5820

gcggtttgcg tattgggcgc tcttccgctt cctcgctcac tgactcgctg cgctcggtcg 5880

ttcggctgcg gcgagcggta tcagctcact caaaggcggt aatacggtta tccacagaat 5940

caggggataa cgcaggaaag aacatgtgag caaaaggcca gcaaaaggcc aggaaccgta 6000

aaaaggccgc gttgctggcg tttttccata ggctccgccc ccctgacgag catcacaaaa 6060

atcgacgctc aagtcagagg tggcgaaacc cgacaggact ataaagatac caggcgtttc 6120

cccctggaag ctccctcgtg cgctctcctg ttccgaccct gccgcttacc ggatacctgt 6180

ccgcctttct cccttcggga agcgtggcgc tttctcatag ctcacgctgt aggtatctca 6240

gttcggtgta ggtcgttcgc tccaagctgg gctgtgtgca cgaacccccc gttcagcccg 6300

accgctgcgc cttatccggt aactatcgtc ttgagtccaa cccggtaaga cacgacttat 6360

cgccactggc agcagccact ggtaacagga ttagcagagc gaggtatgta ggcggtgcta 6420

cagagttctt gaagtggtgg cctaactacg gctacactag aaggacagta tttggtatct 6480

gcgctctgct gaagccagtt accttcggaa aaagagttgg tagctcttga tccggcaaac 6540

aaaccaccgc tggtagcggt ggtttttttg tttgcaagca gcagattacg cgcagaaaaa 6600

aaggatctca agaagatcct ttgatctttt ctacggggtc tgacgctcag tggaacgaaa 6660

actcacgtta agggattttg gtcatgagat tatcaaaaag gatcttcacc tagatccttt 6720

taaattaaaa atgaagtttt aaatcaatct aaagtatata tgagtaaact tggtctgaca 6780

gttaccaatg cttaatcagt gaggcaccta tctcagcgat ctgtctattt cgttcatcca 6840

tagttgcctg actccccgtc gtgtagataa ctacgatacg ggagggctta ccatctggcc 6900

ccagtgctgc aatgataccg cgagacccac gctcaccggc tccagattta tcagcaataa 6960

accagccagc cggaagggcc gagcgcagaa gtggtcctgc aactttatcc gcctccatcc 7020

agtctattaa ttgttgccgg gaagctagag taagtagttc gccagttaat agtttgcgca 7080

acgttgttgc cattgctaca ggcatcgtgg tgtcacgctc gtcgtttggt atggcttcat 7140

tcagctccgg ttcccaacga tcaaggcgag ttacatgatc ccccatgttg tgcaaaaaag 7200

cggttagctc cttcggtcct ccgatcgttg tcagaagtaa gttggccgca gtgttatcac 7260

tcatggttat ggcagcactg cataattctc ttactgtcat gccatccgta agatgctttt 7320

ctgtgactgg tgagtactca accaagtcat tctgagaata gtgtatgcgg cgaccgagtt 7380

gctcttgccc ggcgtcaata cgggataata ccgcgccaca tagcagaact ttaaaagtgc 7440

tcatcattgg aaaacgttct tcggggcgaa aactctcaag gatcttaccg ctgttgagat 7500

ccagttcgat gtaacccact cgtgcaccca actgatcttc agcatctttt actttcacca 7560

gcgtttctgg gtgagcaaaa acaggaaggc aaaatgccgc aaaaaaggga ataagggcga 7620

cacggaaatg ttgaatactc atactcttcc tttttcaata ttattgaagc atttatcagg 7680

gttattgtct catgagcgga tacatatttg aatgtattta gaaaaataaa caaatagggg 7740

ttccgcgcac atttccccga aaagtgccac ctgacgtcta agaaaccatt attatcatga 7800

cattaaccta taaaaatagg cgtatcacga ggccctttcg tctcgcgcgt ttcggtgatg 7860

acggtgaaaa cctctgacac atgcagctcc cggagacggt cacagcttgt ctgtaagcgg 7920

atgccgggag cagacaagcc cgtcagggcg cgtcagcggg tgttggcggg tgtcggggct 7980

ggcttaacta tgcggcatca gagcagattg tactgagagt gcaccatatg ccaccgtgag 8040

ctgcacctga atcagctgag aatgccctga accctcaaac ccgcaccctg aacctcaacc 8100

ttgcgttgaa cccgaggggt gcgggttgga cgcgcacccc gaccactgca ccccgcgggg 8160

cgcccctgtg acgaccatgg tctgattcac gcctgaaatc actccccgcc ggggtggaga 8220

accacgtcaa cgcggccgtg gatcacatcg ggcgtcgaaa aacaaccccc catttcccca 8280

accctcaacc tgatcctgca ctgttgtcgg gtttgctgag agccgcctaa gctgccgcac 8340

gttgtcccag ttggggcgtg gcctgctgca tacggggccg ggaaagacgc ctcacctggg 8400

atgacgcgga ccattggaca cggcctttcc ggccgcggga aggaccagac gtgaatccct 8460

tcgtcaagac ggcgcgcgtg gctatcacct cgacgctggt ggcaggctcg ctcgccactg 8520

ccagcctcgt gtttgcacca cttgcacagg ccgaaagctt agtgagagaa agaggtcctc 8580

agagagtagc agctcacata actgggacca gaggaagaag caacacattg tcttctccaa 8640

actccaagaa tgaaaaggct ctgggccgca aaataaactc ctgggaatca tcaaggagtg 8700

ggcattcatt cctgagcaac ttgcacttga ggaatggtga actggtcatc catgaaaaag 8760

ggttttacta catctattcc caaacatact ttcgatttca ggaggaaata aaagaaaaca 8820

caaagaacga caaacaaatg gtccaatata tttacaaata cacaagttat cctgacccta 8880

tattgttgat gaaaagtgct agaaatagtt gttggtctaa agatgcagaa tatggactct 8940

attccatcta tcaaggggga atatttgagc ttaaggaaaa tgacagaatt tttgtttctg 9000

taacaaatga gcacttgata gacatggacc atgaagccag ttttttcggg gcctttttag 9060

ttggctaact gca 9073

<210> 43
<211> 205
<212> PRT
<213> artificial

<220>
<223> Fusion protein sequence encoded by pFB4:TRAIL

<400> 43

Met Asn Pro Phe Val Lys Thr Ala Arg Val Ala Ile Thr Ser Thr Leu
1               5                   10                  15

Val Ala Gly Ser Leu Ala Thr Ala Ser Leu Val Phe Ala Pro Leu Ala
            20                  25                  30

Gln Ala Glu Ser Leu Val Arg Glu Arg Gly Pro Gln Arg Val Ala Ala
        35                  40                  45

His Ile Thr Gly Thr Arg Gly Arg Ser Asn Thr Leu Ser Ser Pro Asn
    50                  55                  60

Ser Lys Asn Glu Lys Ala Leu Gly Arg Lys Ile Asn Ser Trp Glu Ser
65                  70                  75                  80

Ser Arg Ser Gly His Ser Phe Leu Ser Asn Leu His Leu Arg Asn Gly
                85                  90                  95

Glu Leu Val Ile His Glu Lys Gly Phe Tyr Tyr Ile Tyr Ser Gln Thr
            100                 105                 110

Tyr Phe Arg Phe Gln Glu Glu Ile Lys Glu Asn Thr Lys Asn Asp Lys
        115                 120                 125

Gln Met Val Gln Tyr Ile Tyr Lys Tyr Thr Ser Tyr Pro Asp Pro Ile
    130                 135                 140

Leu Leu Met Lys Ser Ala Arg Asn Ser Cys Trp Ser Lys Asp Ala Glu
145                 150                 155                 160

Tyr Gly Leu Tyr Ser Ile Tyr Gln Gly Gly Ile Phe Glu Leu Lys Glu
                165                 170                 175

Asn Asp Arg Ile Phe Val Ser Val Thr Asn Glu His Leu Ile Asp Met
            180                 185                 190

Asp His Glu Ala Ser Phe Phe Gly Ala Phe Leu Val Gly
        195                 200                 205

<210>  44
<211>  1719
<212>  DNA
<213>  Propionibacterium freudenreichii

<400>  44
gtgaatccct tcgtcaagac ggcgcgcgtg gctatcacct cgacgctggt ggcaggctcg      60

ctcgccactg ccagcctcgt gtttgcacca cttgcacagg ccgattactc cccccttgcg     120

gccaccgcca cggtgaatgt gcgccagggc cccgacacct ccagctcggt gctggcgacg     180

ctctcgtcgg gtgacacggt cacccagcgg ggagccgaac aggacggctg gctgccgatc     240

acctacaacg gcgccaacgc gtggatccag gcgcagtacg tggcctccac cacggccgcc     300

acccagaagg accagatctc caccgccgag ctcacggccg atgcctatgt gcgcaccgcg     360

gccaatgcga acgcctgggt gttgggcacg gcccacaccg gcgacaaggt gggcatcacc     420

ggccaggcca gcggcgacta cacgccggtg aacttctacg gccgcgccgg ttggatcgcc     480

accaagctgc tcagcgcggc cgacgcctcg gtgacatcga tcaagatcac caccgccatc     540

tcgagcgact acctgtgggt gcgtggtggc gagagcaccg cggcccagtc catcggcatg     600

ctctacccgg gtgaccgggt ggacgtgacc ggcgatcccg tcggcgggtg ggtgccgatc     660

aacttcaacg gcaagaccgc cttcgtggcc gcgaactact cgcgctatct cactgatccc 720

accgtggtca cgctgtcgac gaagaccgac gtcaccaaca aggacacggc cacctcgacg 780

ggcaccgatt cctcgacggc cggcggctcg accgccacca ccccgaccac cacggcgcca 840

accaccaccg ctcctgcaac caagccgacg accacgcccc cggccacgac gcaggccgct 900

gcgtccacga agtacacgac ggccgacgtc aacgtgcgcg tgggacccgg catcgaccag 960

cagccggtga cggtgctcaa ggagaactcg caggtggccg ccaccggcaa gacaagtggc 1020

gactggaccg aggtcagcta cgacggcgcc tcgcgctgga tcagcagcca gtacctctcg 1080

gacaccaagc aggccgaggc acccgccccg gcacctgcgc ccgatccgac gcccgccggc 1140

cccaccggaa gccggtggac cacggcggca ctgaacgcct atggcagctc cacccagccc 1200

aagccggcca ccacggtggt gcccgagggc acccaggtgg aactgaccgg caagcaggcc 1260

gatggacgct cggagtacac gtggaacggc acgacctatt ggtcggccac cgaatacctc 1320

ggcaccaatg cgccggccac gaacacctca gcgaacaccg ccaagccggg cgccaacgcg 1380

gtggagacgg cgatcaactt cgcgatgtcg aagctcggtg gcccctatgt ctggggcggc 1440

accggtccgg tgggctatga ctgctccgga ctgatgcagg ccgcgtacgc ggcggccggc 1500

gtcaccctgc cgcgcgtcac ctgggaccag gtgaatgccg gcaagcaggt gtcggtcggc 1560

gacctgcagc cgggcgacct ggtgttcttc tatgacaacg gccacgtggg catgtacatc 1620

ggcaacggca acatcgtcaa cgccctcaac gaggacgccg gcatcgtggt gaccccgatc 1680

agctatatgc cgatctcggc tgccgtccgg atcgcctga 1719

<210> 45
<211> 26
<212> PRT
<213> Propionibacterium acnes

<400> 45

Met Ser Lys Val Val Ala Ser Ala Ile Ala Gly Ala Leu Ser Leu Thr
1               5                   10                  15

Ser Ala Gly Gly Leu Thr Met Val Gln Ala
            20                  25

<210> 46
<211> 30
<212> PRT
<213> Propionibacterium acnes

<400> 46

Met Arg Lys Ala Ile Val Thr Pro Val Ala Val Leu Ala Val Leu Val
1               5                   10                  15

Met Ala Leu Thr Gly Cys Gly Gln Lys Asn Gln Ser Gly Gly
            20                  25                  30

<210>  47
<211>  29
<212>  PRT
<213>  Propionibacterium acnes

<400>  47

Met Ala Ser Thr Pro Arg Arg Arg Trp Ala Trp Val Leu Leu Leu Val
1               5                   10                  15

Val Ala Ser Leu Val Ile Val Gly Val Tyr Arg Lys Ala
            20                  25

<210>  48
<211>  26
<212>  PRT
<213>  Propionibacterium acnes

<400>  48

Met Ser Ser Met Lys Gly Leu Ser Leu Val Leu Ala Thr Ser Phe Met
1               5                   10                  15

Leu Ser Phe Ser Pro Gly Ser Ser Phe Ala
            20                  25

<210>  49
<211>  44
<212>  PRT
<213>  Propionibacterium acnes

<400>  49

Met Glu His Arg Tyr Gly Ala Ser Gln Val Ser Gly Ser Ala Pro Arg
1               5                   10                  15

Arg Gly Arg Gly Val Ala Phe Ala Ala Ile Thr Gly Ala Ile Leu Leu
            20                  25                  30

Gly Thr Val Ala Ser Val Asp Pro Gly Ala Gln Ala
        35                  40

<210>  50
<211>  27
<212>  PRT
<213>  Propionibacterium acnes

<400>  50

Met Ser Ser Met Lys Gly Leu Ser Leu Val Leu Ala Thr Ser Phe Met
1               5                   10                  15

Leu Ser Phe Ser Pro Gly Ser Ser Phe Ala Ser
            20                  25

<210>  51
<211>  47
<212>  PRT
<213>  Propionibacterium acnes

<400>  51

Met Pro His Ser Asp Gln Pro Thr Ser Lys Arg Val Met Ser Ala Pro
1               5                   10                  15

Arg Arg Arg Met Pro Gly Trp Val Pro Val Thr Val Gly Ile Ala Val
            20                  25                  30

Val Val Ile Val Val Val Ala Val Ile Val Ser Ser Leu Arg Ser
        35                  40                  45

<210>  52
<211>  32
<212>  PRT
<213>  Propionibacterium acnes

<400>  52

Met Phe Gly Thr Pro Ser Arg Arg Thr Phe Leu Thr Ala Ser Ala Leu
1               5                   10                  15

Ser Ala Met Ala Leu Ala Ala Ser Pro Thr Val Thr Asp Ala Ile Ala
            20                  25                  30

<210>  53
<211>  26
<212>  PRT
<213>  Propionibacterium acnes

<400>  53

Met Lys Ile Asn Ala Arg Phe Ala Val Met Ala Ala Ser Val Ala Val
1               5                   10                  15

Leu Met Ala Ala Ala Pro Ile Ala Gln Ala
            20                  25

<210>  54
<211>  50
<212>  PRT
<213>  Propionibacterium acnes

<400>  54

Met Tyr Pro Val His Leu Pro Leu Arg Asn Glu Ser Glu Phe Ser Phe

1               5                   10                  15

Arg Ala His Asn His Gly Gly Thr Val Pro Ser Arg Leu Thr Arg Arg
            20                  25                  30

Ser Val Leu Ala Thr Gly Ala Val Ala Leu Pro Met Thr Ala Ala Ala
        35                  40                  45

Cys Ala
    50

<210>  55
<211>  27
<212>  PRT
<213>  Propionibacterium acnes

<400>  55

Met Arg His Met Arg Pro Leu Ile Ala Leu Ser Leu Ala Gly Leu Met
1               5                   10                  15

Thr Leu Ser Ala Cys Gly Glu Asp Val Ala Ala
            20                  25

<210>  56
<211>  31
<212>  PRT
<213>  Propionibacterium acnes

<400>  56

Met Asn Arg Thr Leu Lys Val Ala Ala Val Gly Ala Ile Ala Ile Leu
1               5                   10                  15

Cys Leu Ala Ala Cys Ser Asp Pro Gly Ser Asp Ser Ala Gln Ser
            20                  25                  30

<210>  57
<211>  24
<212>  PRT
<213>  Propionibacterium acnes

<400>  57

Met Glu Lys Ser Ser Phe Ala Ala Ala Asn Met Thr Ile Met Ser Glu
1               5                   10                  15

Pro Thr Thr Pro Thr Ser Gln Ala
            20

<210>  58
<211>  281
<212>  PRT

<213>  Homo sapiens

<400>  58

<210>  59
<211>  22
<212>  PRT
<213>  artificial

<220>
<223>  Signal peptide of Surface layer protein A of Propionibacterium
       freudenreichii strain CIRM BIA N° 118

<400>  59

Met Ala Thr Gly Ala Ala Ala Ala Met Phe Val Thr Thr Phe Ala Gly
1               5                   10                  15

Met Ala Pro Ala Asn Ala
            20

<210>  60
<211>  29
<212>  PRT
<213>  artificial

<220>
<223>  Signal peptide of Surface layer protein B of Propionibacterium
       freudenreichii strain CIRM BIA N° 118

<400>  60

Met Ser Val Arg Lys Ser Leu Thr Gly Met Ala Leu Gly Leu Ala Leu
1               5                   10                  15

Thr Ile Thr Pro Leu Ala Gly Ala Val Pro Ala Ser Ala
            20                  25

<210>  61
<211>  28
<212>  PRT
<213>  artificial

<220>
<223>  Signal peptide of Surface layer protein D of Propionibacterium
       freudenreichii strain CIRM BIA N° 118

<400>  61

Met Arg Arg Phe Phe Ser Ala Ala Ile Ala Ile Leu Leu Ala Ala Thr

1               5                   10                  15

Leu Thr Pro Ala Leu Asn Ala Pro Met Ala Ser Ala
            20                  25

<210>  62
<211>  28
<212>  PRT
<213>  artificial

<220>
<223>  Signal peptide of Surface layer protein D of Propionibacterium
       freudenreichii strain CIRM BIA N° 121

<400>  62

Met Arg Arg Phe Phe Ser Ala Ala Ile Ala Ile Leu Leu Ala Ala Thr
1               5                   10                  15

Leu Thr Pro Ala Leu Asn Ala Pro Met Ala Ser Ala
            20                  25

<210>  63
<211>  29
<212>  PRT
<213>  artificial

<220>
<223>  Signal peptide of Surface layer protein B of Propionibacterium
       freudenreichii strain CIRM BIA N° 122

<400>  63

Met Ser Val Arg Lys Ser Leu Thr Gly Met Ala Leu Gly Leu Ala Leu
1               5                   10                  15

Thr Ile Thr Pro Leu Ala Gly Ala Val Pro Ala Ala Ala
            20                  25

<210>  64
<211>  28
<212>  PRT
<213>  artificial

<220>
<223>  Signal peptide of Surface layer protein D of Propionibacterium
       freudenreichii strain CIRM BIA N° 122

<400>  64

Met Arg Arg Phe Phe Ser Ala Ala Ile Ala Ile Leu Leu Ala Ala Thr
1               5                   10                  15

Leu Thr Pro Ala Leu Asn Ala Pro Met Ala Ser Ala
            20                  25

<210>  65
<211>  33
<212>  PRT
<213>  artificial

<220>
<223>  Signal peptide of Surface layer protein E of Propionibacterium
       freudenreichii strain CIRM BIA N° 122

<400>  65

Met Lys Thr Arg Val Arg Ser Arg Lys Pro Ala Ala Gly Leu Ala Gly
1               5                   10                  15

Ile Ala Leu Phe Ala Ser Gly Leu Ser Leu Met Ser Thr Val Ala Ser
            20                  25                  30

Arg

<210>  66
<211>  22
<212>  PRT
<213>  artificial

<220>
<223>  Signal peptide of Surface layer protein A of Propionibacterium
       freudenreichii strain CIRM BIA N° 125

<400>  66

Met Ala Thr Gly Ala Ala Ala Ala Met Phe Val Thr Thr Phe Ala Gly
1               5                   10                  15

Met Ala Pro Ala Asn Ala
            20

<210>  67
<211>  22
<212>  PRT
<213>  artificial

<220>
<223>  Signal peptide of Surface layer protein A of Propionibacterium
       freudenreichii strain CIRM BIA N° 129

<400>  67

Met Ala Thr Gly Ala Ala Ala Ala Met Phe Val Thr Thr Phe Ala Gly
1               5                   10                  15

Met Ala Pro Ala Asn Ala
            20

<210>  68

<211>  22
<212>  PRT
<213>  artificial

<220>
<223>  Signal peptide of Surface layer protein A of Propionibacterium
       freudenreichii strain CIRM BIA N° 512

<400>  68

Met Ala Thr Gly Ala Ala Ala Ala Met Phe Val Thr Thr Phe Ala Gly
1               5                   10                  15

Met Ala Pro Ala Asn Ala
            20

<210>  69
<211>  624
<212>  DNA
<213>  artificial

<220>
<223>  DNA fragment from Propionibacterium freudenreichii strain CIRM
       BIA N° 118 comprising the promotor and the signal peptide of the
       Surface layer protein A

<400>  69
gatcccccga ggtccacacg ccgggccgag gcgggtacac ctgccgggca ggccgggggt      60

tccggctctg ggaatggagc tgttcggtta tccaccatgt ggacgacacg caggcaccat     120

gctcgtcttc gagcggaatc acgggccccg ataactgtgt gatttgtgcg tgaaggccga     180

ttcgggggcc gtttgagcgg cgcgccgcac ggataacagc gttctatgcc accgatacac     240

caatagtccc caagtgcttg acaggactct tcgaagcctc tttgaagatg gctctgacta     300

ggggaaacag tgctcagtag tcaccgcgaa ggagcagtgg ctctgttact gttctagcgg     360

ctggacgggt agttcgattg atgatacgct tgcctcggca tagtgggtcg ccaatcctag     420

ggaacccatc gaaggccatg aattttccca gaggacatgg gtgttcgaac atcccggcag     480

agctcttgaa ggtcggccgt gcacccttgt ccaggcttga aaggaaacaa gttgtctgtt     540

cgtaggattg cggtggccac aggtgctgcg gccgccatgt ttgtgacgac gtttgcgggc     600

atggcgcctg cgaatgccaa ggat                                            624

<210>  70
<211>  30
<212>  DNA
<213>  artificial

<220>
<223>  Forward primer PSlpA_fwd_BamHI

<400>  70
atgcggatcc ccccgaggtc cacacgccgg                                       30

<210>  71
<211>  30
<212>  DNA
<213>  artificial

<220>
<223>  Reverse primer PS_SlpA-EcoRV-Rev_bis

<400>  71
atgcgatatc ggcattcgca ggcgccatgc                                        30